# EUROPÄISCHE PATENTSCHRIFT

(11) **EP 1 292 220 B1**
(45) Veröffentlichungstag und Bekanntmachung des Hinweises auf die Patenterteilung: **13.12.2006**
(21) Anmeldenummer: 01951354.8
(22) Anmeldetag: 31.05.2001
(51) Int. Cl.: A61B 5/00, G01N 21/65

(54) **VERFAHREN UND VORRICHTUNG ZUM NACHWEISEN VON SUBSTANZEN IN KÖRPERFLÜSSIGKEITEN MITTELS RAMAN-SPEKTROSKOPIE**
METHOD AND DEVICE FOR DETECTING SUBSTANCES IN BODY FLUIDS BY RAMAN SPECTROSCOPY
PROCEDE ET DISPOSITIF POUR DETECTER DES SUBSTANCES DANS DES LIQUIDES ORGANIQUES PAR SPECTROSCOPIE RAMAN

(30) Priorität: 31.05.2000 DE 10027100
(43) Veröffentlichungstag der Anmeldung: 19.03.2003
(73) Patentinhaber: Müller-Dethlefs, Klaus, York, Yorkshire YO10 4BG (GB)
(72) Erfinder: Müller-Dethlefs, Klaus, York, Yorkshire YO10 4BG (GB)
(74) Vertreter: Habenicht, Wieland
(86) Internationale Anmeldenummer: PCT/DE2001/002068
(87) Internationale Veröffentlichungsnummer: WO 2001/091632

(56) Entgegenhaltungen:
- WO-A-00/02479
- WO-A-92/15008
- WO-A-99/55222
- US-A- 5 515 847
- US-A- 5 553 616
- US-A- 6 070 093

## Beschreibung

Die Erfindung betrifft Verfahren und Vorrichtungen zum nicht-invasiven Bestimmen der Konzentration und Nachweisen von Substanzen in Körperflüssigkeiten mit Raman- Spektroskopie. Insbesondere betrifft die Erfindung den Nachweis und die Konzentrationsbestimmung von Glukose, Cholesterin, Laktat oder dergleichen im Blut des Menschen.

Zum Nachweis von im Blut eines Patienten gelösten Stoffen wird in den meisten Fällen dem Patienten Blut abgenommen und anschließend analysiert. Solange diese Blutuntersuchung nur einmal oder in zeitlich relativ großen Abständen und durch entsprechend geschultes Personal ambulant erfolgt, bedeutet dies für den Patienten keine übermäßig große Belastung. Ist jedoch eine häufige, in regelmäßigen Abständen wiederkehrende Untersuchung des Blutes erforderlich, wie dies bei Diabetes- Patienten der Fall ist, so ist eine häufige Blutabnahme dem Patienten in einer Klinik oder einer Arztpraxis nicht zuzumuten. Daher wurden sogenannte "home monitoring"- Verfahren entwickelt, mit denen sich der Patient selbst kontrollieren kann; und zwar zeitlich wie räumlich unabhängig. Dies setzt jedoch voraus, dass der Patient immer sein dazu erforderliches Besteck mit sich führt. Der generelle Nachteil bei diesen Verfahren ist, dass eine Blutabnahme jedesmal mit einem - wenn auch sehr geringen - Infektionsrisiko behaftet ist. Wenn daher die entsprechenden Geräte (Spritzen etc.) nicht oder nicht steril verfügbar sind, ist eine Blutuntersuchung nicht möglich. Mit noch größeren Schwierigkeiten verbunden ist die Blutuntersuchung mit Blutabnahme bei hämophil veranlagten Patienten. In jedem Fall bedeutet die Analyse der Blutwerte mit Blutabnahme für den Patienten einen mehr oder minder schmerzhaften Eingriff und einen Aufwand, dem er selbst nicht immer und ohne weiteres gewachsen ist.

Das in einer Klinik oder Arztpraxis ambulant abgenommene Blut muss in einem Labor (nass- ) chemisch untersucht werden. Bei den "home monitoring"- Verfahren hat der Patient dagegen eine trockenchemische Ausrüstung (Minilabor), häufig in Form von Teststreifen etc., meistens mit der Blutabnahme in einem Gerät integriert, so dass sich die Bearbeitung in einem eigens dafür eingerichteten Labor erübrigt. Der Nachteil jeder chemischen Untersuchung besteht jedoch in den hohen Anforderungen, die an Sauberkeit und Dosiergenauigkeit gestellt werden müssen. Außerdem besteht bei allen in vitro Verfahren die Gefahr, dass Blut und/oder Chemikalien in die Umgebung gelangen und damit u. U. auch Krankheitserreger verbreitet werden können.

Die für den Patienten notwendige Blutentnahme, oft mehrmals am Tag, stellt eine große Belastung für den Patienten dar, sowohl gesundheitlich und psychisch als auch durch die Einschränkung seiner Mobilität.

Aus diesem Grunde wurden Methoden entwickelt, die eine Untersuchung des Patientenblutes in vivo ohne Abnahme von Blut ermöglichen und die die Angabe des Ergebnisses ohne nennenswerte Zeitverzögerung - was sich bei der chemischen Analyse nicht vermeiden lässt - zulassen. Insbesondere wurden Verfahren zur Bestimmung der Konzentration von medizinisch relevanten Stoffen in der Körperflüssigkeit eines Patienten beschrieben, die auf den durch die nachzuweisenden Stoffe veränderten physikalischen Eigenschaften von Licht beruhen.

Aus DE- A- 195 18 511 ist ein Verfahren für die transcutane, unblutige Konzentrationsbestimmung von Substanzen im Blut des Menschen bekannt. Bei der transcutanen, unblutigen in- vivo-Konzentrationsbestimmung von im Patientenblut zu bestimmenden Substanzen wie Glucose, Lactat Blutzucker, Cholesterin, Alkohol, Drogen oder dergleichen wird ein der Menge einer Substanz und der Wassermenge einer gegebenen Körperregion entsprechendes Signal, das mittels spektroskopischer Methoden erzeugt wird, gemessen, die Konzentration im Wasser durch Verhältnisbildung des Signalwertes der Substanz- und Wassermenge ermittelt und hieraus der Blutkonzentrationswert errechnet. Insbesondere wird als spektroskopische Methode die Kernspinresonanzspektroskopie und neben anderen spektroskopischen Methoden in allgemeinem Zusammenhang auch die Raman- Spektroskopie genannt.

Einer Anwendung der Raman- Spektroskopie zur Konzentrationsmessung von Substanzen in Gewebeflüssigkeiten stehen jedoch schwerwiegende Probleme entgegen. Die Intensität der Raman-Streuung ist nur klein und generell gegenüber der Rayleighstreuung um einige Größenordnungen geringer. Für menschliches Gewebe kann die Rayleighstreuung (unter der hier alle Streuprozesse, die die gestreute Wellenlänge nicht verändern, zusammengefasst werden, also auch die Partikelstreuung) wegen der inhomogenen und opaken Eigenschaften des Mediums sogar um etwa 10 Größenordnungen größer als die Raman- Streuung sein. Diese starke Rayleighstreuung "blendet" bekannte Detektionssysteme im Bereich der wellenlängenverschobenen Raman- Streuung. Neben der Rayleighstreuung kann in menschlichem Gewebe je nach Anregungswellenlänge auch unerwünschte Fluoreszenz oder andere störende Lichtemission auftreten, die die Raman- Signale überdeckt. Ein weiteres Problem ergibt sich aus der spektralen Überlagerung von Raman- Signalen verschiedener anderer Substanzen mit dem Signal der nachzuweisenden Substanz. Aufgrund der komplexen Zusammensetzung des Mediums ergeben sich Störsignale die wesentlich größer als die Messsignale für die nachzuweisende Substanz sein können.

Aus DE 691 21 589 T2 ist ein nicht- invasives Verfahren zur Messung der Konzentration von D- Glucose im Augenkammerwasser mit Raman- Spektroskopie sowie eine Vorrichtung dafür bekannt.

Aus DE 195 38 372 A1 ist ein Verfahren und eine Vorrichtung ebenfalls für eine nicht- invasive Glukosemessung im Auge mit Raman- Spektroskopie bekannt.

Aus DE 692 19 580 T2 ist die Bestimmung der Zusammensetzung und der Konzentration eines arbiträren Gasgemisches in dem Luftweg eines Patienten mittels Raman- Spektroskopie bekannt.

In WO-A-0 002 479 wird ein nicht-invasives Glukose-Messgerät für das Auge beschrieben. Das Auge als Untersuchungsobjekt hat den Vorteil, dass der untersuchte Glaskörper keine lichtstreuenden Partikel enthält und optisch sehr homogen ist. Außerdem sind Druck und Dichte des Glaskörpers konstant, d.h. der Kreislauf hat keinerlei Einfluss auf die Messung. Schließlich ist der optisch erfasste Beobachtungsraum wohl definiert, was die Messung sehr vereinfacht. Diese Vorteile sind aber bei Messungen an anderen Körperteilen nicht gegeben. Daher kann das Verfahren nicht ohne weiteres für die nicht-invasive Untersuchung an beliebigen, leichter zugänglichen Körperteile angewendet werden. Vielmehr ist dort eine sehr viel größere Streuung von Licht zu erwarten, so dass Aussagen über die Konzentration einer gesuchten Substanz sehr viel schwieriger sind. Ferner hängen die Messergebnisse davon ab, ob sich das Körpergewebe in einer stark oder einer schwächer durchbluteten Region befindet.

In WO-A-9 215 008 werden ein System und ein Verfahren beschrieben, womit zu Zwecken der Diagnose molekulare Spektroskopie an Gewebe durchgeführt werden kann. Die Schrift bezieht sich dabei aber auf entnommene Gewebeproben bzw. auf "in vivo"-Messungen innerhalb des Gewebes, d.h. Messungen, die mit einem Katheter durchgeführt werden. Aufgrund des erheblich höheren statistischen Rauschens und der Abhängigkeit der Messergebnisse von sich zeitlich ändernden Parametern bei nicht-invasiven Verfahren lässt sich das kathetergestütze Verfahren nach dem Stand der Technik nicht ohne weiteres in ein nicht-invasives Verfahren umwandeln.

Der Erfindung liegt daher die Aufgabe zugrunde, Verfahren und Vorrichtungen zum nicht-invasiven Nachweis von Substanzen in Körperflüssigkeiten zu schaffen, womit sich bei sehr guter Analysegenauigkeit eine für den Patienten akzeptable kurze Messdauer einhalten lässt.

Die Aufgabe wird erfindungsgemäß durch Verfahren gemäß Anspruch 1, 2 bzw. 3 und durch Vorrichtungen gemäß Anspruch 18, 19 bzw. 20 gelöst. Vorteilhafte Ausgestaltungen der Erfindung sind Gegenstand der jeweiligen Unteransprüche.

Der Erfindung liegt das Prinzip zugrunde, die Raman- Streuung von Primärlicht an einer nachzuweisenden Substanz zu nutzen, um ein mit der Konzentration der nachzuweisenden Substanz korreliertes Signal zu erhalten. Um Störungen der Messung des Raman-Signals der nachzuweisenden Substanz auszuschließen oder wenigstens zu minimieren, wird erfindungsgemäß das Wellenlängenspektrum des Sekundärlichtes im Bereich des Raman- Spektrums der nachzuweisenden Substanz für zwei verschiedene Primärlichtwellenlängen aufgenommen. Nach Erkenntnis des Erfinders ist das Raman- Spektrum der nachzuweisenden Substanz entsprechend der unterschiedlichen Primärlichtwellenlänge verschoben, das Raman-Spektrum des Körpergewebes für beide Primärlichtwellenlängen aber weitestgehend identisch. Durch Vergleich der Raman- Spektren bei der ersten und bei der zweiten Primärlichtwellenlänge lässt sich das Hintergrundsignal des Körpergewebes eliminieren und ein Signal gewinnen, welches proportional zu der Konzentration der nachzuweisenden Substanz ist.

Gegenstand der Erfindung ist ein Verfahren zum nicht- invasiven Bestimmen einer Konzentration einer Substanz in einer Körperflüssigkeit in einem Körpergewebe mit Raman- Spektroskopie, das die Schritte umfasst: a) Einstrahlen von monochromatischem Primärlicht einer ersten Wellenlänge λ₁ in das Körpergewebe, b) Erfassen von Sekundärlicht, das von dem Körpergewebe zurückgestreut wird, und Abspeichern der Intensität des Sekundärlichts in Abhängigkeit von der Wellenlänge als erstes Raman- Spektralsignal bei der ersten Wellenlänge in einem ersten Speicher, c) Einstrahlen von monochromatischem Primärlicht einer zweiten Wellenlänge λ₂ in das Körpergewebe, d) Erfassen von Sekundärlicht, das von dem Körpergewebe zurückgestreut wird, und Abspeichern der Intensität des Sekundärlichts in Abhängigkeit von der Wellenlänge als zweites Raman- Spektralsignal bei der zweiten Wellenlänge in einem zweiten Speicher, e) Vergleichen des ersten Raman- Spektralsignals in dem ersten Speicher und des zweiten Raman- Spektralsignals in dem zweiten Speicher miteinander und Erzeugen eines Vergleichssignals, bei dem spektrale Strukturen, die durch das Körpergewebe bedingt sind, im wesentlichen vollständig eliminiert sind, f) Bestimmen einer Intensität des Vergleichssignals bei wenigstens einer Abfragewellenlänge λ₃, wobei die Intensität der Konzentration der Substanz in der Körperflüssigkeit entspricht, wobei das Primärlicht der ersten und/oder zweiten Wellenlänge mit einer vorgegebenen Frequenz eingestrahlt wird und das erste und das zweite Raman-Spektralsignal mit der vorgegebenen Frequenz aufgenommen wird.

Ein weiterer Gegenstand der Erfindung ist ein Verfahren zum nicht- invasiven Bestimmen einer Konzentration einer Substanz in einer Körperflüssigkeit in einem Körpergewebe mit Raman-Spektroskopie, das die Schritte umfasst: a) Einstrahlen von monochromatischem Primärlicht einer vorgegebenen Wellenlänge λ in das Körpergewebe bei einer ersten Temperatur des Körpergewebes, b) Erfassen von Sekundärlicht, das von dem Körpergewebe zurückgestreut wird, und Abspeichern der Intensität des Sekundärlichts in Abhängigkeit von der Wellenlänge als erstes Raman- Spektralsignal bei der ersten Temperatur in einem ersten Speicher, c) Einstrahlen von monochromatischem Primärlicht der vorgegebenen Wellenlänge λ in das Körpergewebe bei einer zweiten Temperatur des Körpergewebes, d) Erfassen von Sekundärlicht, das von dem Körpergewebe zurückgestreut wird, und Abspeichern der Intensität des Sekundärlichts in Abhängigkeit von der Wellenlänge als zweites Raman- Spektralsignal bei der zweiten Temperatur in einem zweiten Speicher, e) Vergleichen des ersten Raman- Spektralsignals in dem ersten Speicher und des zweiten Raman- Spektralsignals in dem zweiten Speicher miteinander und Erzeugen eines Vergleichssignals, bei dem spektrale Strukturen, die durch das Körpergewebe bedingt sind, im wesentlichen vollständig eliminiert sind, f) Bestimmen einer Intensität des Vergleichssignals bei wenigstens einer Abfragewellenlänge λ₃, wobei die Intensität der Konzentration der Substanz in der Körperflüssigkeit entspricht.

Ein weiterer Gegenstand der Erfindung ist ein Verfahren zum nicht- invasiven Nachweis einer Substanz in einer Körperflüssigkeit in einem Körpergewebe mit Raman- Spektroskopie, das die Schritte umfasst: a) Einstrahlen von monochromatischem Primärlicht einer ersten Wellenlänge λ₁ in das Körpergewebe bei einer ersten Temperatur, b) Erfassen von Sekundärlicht, das von dem Körpergewebe zurückgestreut wird, und Abspeichern der Intensität des Sekundärlichts in Abhängigkeit von der Wellenlänge als erstes Raman- Spektralsignal bei der ersten Wellenlänge und bei der ersten Temperatur in einem ersten Speicher, c) Einstrahlen von monochromatischem Primärlicht einer zweiten Wellenlänge λ₂ in das Körpergewebe bei einer ersten Temperatur, d) Erfassen von Sekundärlicht, das von dem Körpergewebe zurückgestreut wird, und Abspeichern der Intensität des Sekundärlichts in Abhängigkeit von der Wellenlänge als zweites Raman- Spektralsignal bei der zweiten Wellenlänge und bei der ersten Temperatur in einem zweiten Speicher, e) Vergleichen des ersten Raman-Spektralsignals in dem ersten Speicher und des zweiten Raman-Spektralsignals in dem zweiten Speicher miteinander und Erzeugen eines ersten Vergleichssignals, bei dem spektrale Strukturen, die durch das Körpergewebe bedingt sind, im wesentlichen vollständig eliminiert sind, f) Bestimmen einer Intensität des ersten Vergleichssignals bei wenigstens einer Abfragewellenlänge λ₃, g) Wiederholen der Schritte a) bis f) bei einer zweiten Temperatur und Erzeugen eines zweiten Vergleichssignals bei der wenigstens einen Abfragewellenlänge λ₃, wobei die Intensität des Sekundärlichtes bei der ersten und der zweiten Wellenlänge in einem dritten Speicher bzw. einem vierten Speicher abgespeichert wird und ein zweites Vergleichssignal erzeugt wird, h) Vergleichen des ersten und des zweiten Vergleichssignals und Erzeugen eines Identifikationssignals in Abhängigkeit von dem Vergleichsergebnis bei der wenigstens einen Abfragewellenlänge λ₃.

Bevorzugte Ausführungsformen der Verfahren zeichnen sich dadurch aus, dass sie eines oder mehrere der folgenden Merkmale aufweisen:
die vorgegebene Frequenz liegt im Bereich von einigen Kilohertz;
die erste und die zweite Temperatur des Körpergewebes wird mit einer vorgegebenen Frequenz eingestellt;
die vorgegebene Frequenz ist die Herzfrequenz;
das Primärlicht mit der ersten und/oder zweiten Wellenlänge weist eine Pulslänge im Pikosekundenbereich auf;
das Primärlicht wird durch einen Laser erzeugt;
das Sekundärlicht wird im Stokes- Bereich und/oder Anti- Stokes- Bereich des Raman- Spektrums erfasst;
die Wellenlängen λ₁ und λ₂ des Primärlichts liegen zwischen 750nm und 850nm;
der Absolutwert der Differenz zwischen der Wellenlänge λ₁ bzw. λ₂ des Primärlichts und der Abfragewellenlänge λ₃ ist kleiner als 250meV (2000cm⁻¹);
der Absolutwert der Differenz zwischen der Wellenlänge λ₁ bzw. λ₂ des Primärlichts und der Abfragewellenlänge λ₃ ist größer als 2, 5meV (20cm⁻¹);
mindestens ein notch- Filter wird zum Eliminieren von Rayleigh- Streuung verwendet;
mit dem Primärlicht wird im wesentlichen zeitgleich ein Anregungslicht mit einer Wellenlänge λ_{Anr} in das Körpergewebe eingestrahlt, die von der Substanz absorbiert wird;
die Wellenlänge λ_{Anr} des Anregungslichtes liegt zwischen 1,2µm und 3µm.

Für die Durchführung der Verfahren werden erfindungsgemäß die folgenden Vorrichtungen geschaffen.

Eine erste Vorrichtung zum nicht- invasiven Bestimmen einer Konzentration einer Substanz in einer Körperflüssigkeit in einem Körpergewebe mit Raman- Spektroskopie ist ausgestattet mit: a) einer ersten Lichtquelle zum Einstrahlen von monochromatischem Primärlicht einer ersten Wellenlänge λ₁ in das Körpergewebe, b) einer Photodetektoreinrichtung zum Erfassen von Sekundärlicht, das von dem Körpergewebe zurückgestreut wird, c) einem ersten Speicher zum Abspeichern der Intensität des Sekundärlichts in Abhängigkeit von der Wellenlänge als därlichts in Abhängigkeit von der Wellenlänge als erstes Raman-Spektralsignal bei der ersten Wellenlänge λ₁, d) einer zweiten Lichtquelle zum Einstrahlen von monochromatischem Primärlicht einer zweiten Wellenlänge λ₂ in das Körpergewebe, e) einem zweiten Speicher zum Abspeichern der Intensität des Sekundärlichts in Abhängigkeit von der Wellenlänge als zweites Raman- Spektralsignal bei der zweiten Wellenlänge λ₂, f) einer Komparatoreinrichtung zum Vergleichen des ersten Raman- Spektralsignals in dem ersten Speicher und des zweiten Raman- Spektralsignals in dem zweiten Speicher miteinander und zum Erzeugen eines Vergleichssignals, bei dem spektrale Strukturen, die durch das Körpergewebe bedingt sind, im wesentlichen vollständig eliminiert sind, g) einer Diskriminatoreinheit zum Bestimmen einer Intensität des Vergleichssignals bei wenigstens einer Abfragewellenlänge λ₃, wobei die Intensität der Konzentration der Substanz in der Körperflüssigkeit entspricht, wobei die erste und/oder zweite Lichtquelle, die Photodetektoreinrichtung und der erste und der zweite Speicher mit einer vorgegebenen Frequenz pulsbar sind.

Eine alternative Vorrichtung zum nicht- invasiven Bestimmen einer Konzentration einer Substanz in einer Körperflüssigkeit in einem Körpergewebe mit Raman- Spektroskopie ist ausgestattet mit: a) einer Lichtquelle zum Einstrahlen von monochromatischem Primärlicht einer vorgegebenen Wellenlänge λ in das Körpergewebe, b) einer Heizeinrichtung zum Einstellen einer ersten Temperatur und einer zweiten Temperatur in dem Körpergewebe, c) einer Photodetektoreinrichtung zum Erfassen von Sekundärlicht, das von dem Körpergewebe zurückgestreut wird, d) einem ersten Speicher zum Abspeichern der Intensität des Sekundärlichts in Abhängigkeit von der Wellenlänge als erstes Raman- Spektralsignal bei der ersten Temperatur und einem zweiten Speicher zum Abspeichern der Intensität des Sekundärlichts in Abhängigkeit von der Wellenlänge als zweites Raman- Spektralsignal bei der zweiten Temperatur, e) einer Komparatoreinrichtung zum Vergleichen des ersten Raman- Spektralsignals in dem ersten Speicher und des zweiten Raman- Spektralsignals in dem zweiten Speicher miteinander und zum Erzeugen eines Vergleichssignals, bei dem spektrale Strukturen, die durch das Körpergewebe bedingt sind, im wesentlichen vollständig eliminiert sind, f) einer Diskriminatoreinrichtung zum Bestimmen einer Intensität des Vergleichssignals bei wenigstens einer Abfragewellenlänge λ₃, wobei die Intensität der Konzentration der Substanz in der Körperflüssigkeit entspricht.

Eine weitere alternative Vorrichtung zum nicht- invasiven Nachweis einer Substanz in einer Körperflüssigkeit in einem Körpergewebe mit Raman- Spektroskopie ist ausgestattet mit: a) einer ersten Lichtquelle zum Einstrahlen von monochromatischem Primärlicht einer ersten Wellenlänge λ₁ in das Körpergewebe, b) einer zweiten Lichtquelle zum Einstrahlen von monochromatischem Primärlicht einer zweiten Wellenlänge λ₂ in das Körpergewebe, c) einer Heizeinrichtung zum Einstellen einer ersten Temperatur und einer zweiten Temperatur in dem Körpergewebe, d) einer Photodetektoreinrichtung zum Erfassen von Sekundärlicht, das von dem Körpergewebe zurückgestreut wird, e) einem ersten Speicher zum Abspeichern der Intensität des Sekundärlichts in Abhängigkeit von der Wellenlänge als erstes Raman- Spektralsignal bei der ersten Wellenlänge λ₁ und bei der ersten Temperatur, einem zweiten Speicher zum Abspeichern der Intensität des Sekundärlichts in Abhängigkeit von der Wellenlänge als zweites Raman-Spektralsignal bei der zweiten Wellenlänge λ₂ und bei der ersten Temperatur, einem dritten Speicher zum Abspeichern der Intensität des Sekundärlichts in Abhängigkeit von der Wellenlänge als drittes Raman- Spektralsignal bei der ersten Wellenlänge λ₁ und bei der zweiten Temperatur und einem vierten Speicher zum Abspeichern der Intensität des Sekundärlichts in Abhängigkeit von der Wellenlänge als viertes Raman- Spektralsignal bei der zweiten Wellenlänge λ₂ und bei der zweiten Temperatur, f) einer ersten Komparatoreinrichtung zum Vergleichen des ersten Raman-Spektralsignals in dem ersten Speicher und des zweiten Raman-Spektralsignals in dem zweiten Speicher miteinander und zum Erzeugen eines ersten Vergleichssignals, bei dem spektrale Strukturen, die durch das Körpergewebe bedingt sind, im wesentlichen vollständig eliminiert sind, g) einer zweiten Komparatoreinrichtung zum Vergleichen des dritten Raman- Spektralsignals in dem dritten Speicher und des vierten Raman- Spektralsignals in dem vierten Speicher miteinander und zum Erzeugen eines zweiten Vergleichssignals, bei dem spektrale Strukturen, die durch das Körpergewebe bedingt sind, im wesentlichen vollständig eliminiert sind, h) einer dritten Komparatoreinrichtung zum Vergleichen des ersten Vergleichssignals und zweiten Vergleichssignals miteinander und zum Erzeugen eines dritten Vergleichssignals in Abhängigkeit von dem Vergleichsergebnis, i) einer Diskriminatoreinrichtung zum Bestimmen einer Intensität des dritten Vergleichssignals bei wenigstens einer Abfragewellenlänge λ₃, j) einer Ausgabeeinheit zum Ausgeben eines Identifikationssignals in Abhängigkeit von der Intensität bei der wenigstens einen Abfragewellenlänge λ₃.

Bevorzugte Ausführungsformen der Vorrichtungen zeichnen sich dadurch aus, dass sie eines oder mehrere der folgenden Merkmale aufweisen:
die erste und/oder zweite Lichtquelle, die Photodetektoreinrichtung und der erste bzw. dritte und/oder zweite bzw. vierte Speicher wird/werden mit einer vorgegebenen Frequenz gepulst;
die vorgegebene Frequenz liegt im Bereich von einigen Kilohertz;
die Heizvorrichtung zum Einstellen der ersten und zweiten Temperatur des Körpergewebes wird mit einer vorgegebenen Frequenz angesteuert;
eine Aufnahmevorrichtung ist zum Erfassen der Herzfrequenz vorgesehen und die vorgegebene Frequenz ist die Herzfrequenz;
die erste und/oder zweite Lichtquelle erzeugt Pulse mit einer Pulslänge im Pikosekundenbereich;
die erste und/oder zweite Lichtquelle ist ein Laser;
die erste und/oder zweite Lichtquelle erzeugt eine Wellenlänge λ₁ bzw. λ₂ des Primärlichtes zwischen 750nm und 850nm;
mindestens ein notch- Filter ist zum Eliminieren von Rayleigh- Streuung zwischen der Abbildungsoptik und der Photodetektoreinrichtung angeordnet;
eine dritte Lichtquelle ist zum Erzeugen von Anregungslicht mit einer Wellenlänge λ_{Anr}, die von der Substanz absorbiert wird, vorgesehen;
die dritte Lichtquelle erzeugt eine Wellenlänge λ_{Anr} zwischen 1,2µm und 3µm;
die dritte Lichtquelle ist ein Laser für die Erzeugung von Pikosekunden.

Einer von mehreren Vorteilen der erfindungsgemäßen Verfahren und Vorrichtungen besteht darin, dass eine zeitlich kontinuierliche statt einer nur diskreten Überwachung der Blutwerte möglich ist. Ferner wird die permanente Verletzung des Gewebes des Patienten durch die Blutabnahme und eine damit möglicherweise einhergehende Entzündung des Gewebes sowie gesteigerte Infektionsgefahr vermieden. Mit den erfindungsgemäßen selektiveren, größere Signalstärken produzierenden Verfahren und Vorrichtungen mit besserem Signalrauschverhältnis lassen sich sehr gute reproduzierbare Analysegenauigkeiten in kurzer Messdauer erzielen.

Weitere Vorteile, Merkmale und Anwendungsmöglichkeiten der vorliegenden Erfindung ergeben sich aus der nachfolgenden Beschreibung von Ausführungsbeispielen, bei der Bezug auf die beigefügten Zeichnungen genommen wird.

Fig. 1 zeigt eine Ausführungsform einer erfindungsgemäßen Vorrichtung zur Durchführung des ersten erfindungsgemäßen Verfahrens.

Fig. 2 zeigt eine Ausführungsform einer erfindungsgemäßen Vorrichtung zur Durchführung des zweiten erfindungsgemäßen Verfahrens.

Fig. 3 zeigt eine Ausführungsform einer erfindungsgemäßen Vorrichtung zur Durchführung des dritten erfindungsgemäßen Verfahrens.

Fig. 4A, B und C zeigen ein erstes Spektralsignal bei einer ersten Primärlichtwellenlänge, ein zweites Spektralsignal bei einer zweiten Primärlichtwellenlänge bzw. das Differenzspektrum der beiden Spektralsignale in Fig. 4A und 4B.

Fig. 5A, B und C zeigen ein erstes Spektralsignal bei einer ersten Temperatur, ein zweites Spektralsignal bei einer zweiten Temperatur bzw. das Differenzspektrum der beiden Spektralsignale in Fig. 5A und 5B.

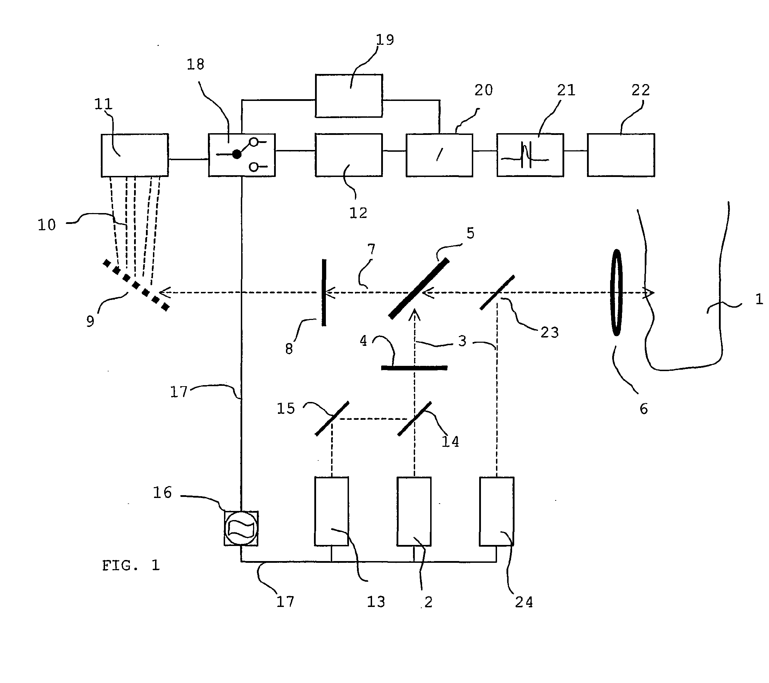

Die Figuren 1, 2 und 3 zeigen den optischen Aufbau nicht maßstabsgetreu.

In Fig. 1 ist eine Ausführungsform des erfindungsgemäßen Grundaufbaus für die Durchführung einer Ausführungsform eines erfindungsgemäßen Verfahrens dargestellt. Die Vorrichtung zum nicht- invasiven Bestimmen einer Konzentration einer Substanz in einer Körperflüssigkeit in einem Körpergewebe 1 mit Raman-Spektroskopie umfasst eine erste Lichtquelle 2, die zum Einstrahlen von monochromatischem Primärlicht 3 einer ersten Wellenlänge λ₁ in das Körpergewebe 1 dient. Das Primärlicht 3 tritt durch ein erstes optisches Filter 4 hindurch, in dem eventuelles Restlicht mit einer anderen Wellenlänge als λ₁ herausgefiltert wird. Dieses Bandpassfilter 4 ist daher insbesondere dann von Vorteil, wenn eine nicht- monochromatische Lichtquelle 2 verwendet wird. Das Primärlicht 3 trifft anschließend auf einen Auskoppelstrahlteiler 5, dessen Funktion weiter unten erläutert wird. Hinter dem Auskoppelstrahlteiler 5 ist eine Abbildungs- / Auffangoptik 6 angeordnet, die das Primärlicht 3 auf eine Stelle des Körpergewebes 1 abbildet. In der dargestellten Ausführungsform wird das Primärlicht 3 auf das Ohrläppchen eines Patienten fokussiert, das rechts in Fig. 1 angedeutet ist.

Das von dem Körpergewebe 1 zurückgestreute Sekundärlicht wird in der gezeigten Ausführungsform der Vorrichtung durch dieselbe Abbildungs- /Auffangoptik 6 aufgenommen. Dies ist besonders vorteilhaft, da nur eine Optik vorgesehen werden muss und quasi automatisch das Sekundärlicht exakt von derselben Stelle des Körpergewebes 1 kommt, die von dem Primärlicht 3 bestrahlt wird. Es ist jedoch auch möglich, die Abbildungsoptik für das Primärlicht 3 und die Auffangoptik für das Sekundärlicht unabhängig voneinander anzuordnen.

Das Sekundärlicht 7 breitet sich über einen kurzen Weg kollinear zu dem Primärlicht 3, aber in entgegengesetzter Richtung aus und tritt durch den Auskoppelstrahlteiler 5 hindurch. In dem Auskoppelstrahlteiler 5 wird das Sekundärlicht von dem Ausbreitungsweg des Primärlichts 3 abgezweigt und breitet sich hinter dem Auskoppelstrahlteiler 5 auf einem Weg 7 weiter aus, der sich von dem Weg des Primärlichtes unterscheidet. Die Trennung von Primärlicht 3 und Sekundärlicht 7 in dem Auskoppelstrahlteiler 5 kann dabei wellenlängenselektiv oder polarisationsselektiv erfolgen, was dem Fachmann allgemein bekannt ist und daher hier nicht weiter erläutert wird. Das Sekundärlicht 7 durchläuft ein zweites optisches Filter 8, in dem unerwünschtes Streulicht herausgefiltert wird. Dieses zweite optische Filter 8 ist ebenso wie das erste optische Bandpassfilter 4 optional. Das Filter 8 dient in erster Linie zum Eliminieren von Rayleigh- Streuung (Streuung ohne Änderung der Wellenlänge).
Als Filter 5 bzw. 8 werden vorzugsweise Notch- Filter verwendet. Bei Notch- Filtern lässt sich die Intensität des durchgelassenen Lichtes aufgrund der winkelabhängigen Reflektivität der Notch- Filter über die Wahl des jeweiligen Eintritts- bzw. Austrittswinkels einstellen. So wird der Eintrittswinkel (und Austrittswinkel) des Auskoppelstrahlteilers 5 vorzugsweise auf 10° eingestellt.

In einem dispergierenden Element 9 (Gitter, Prisma) wird das Sekundärlicht 7 in spektral zerlegtes Sekundärlicht 10 aufgespalten, das seinerseits von einer Photodetektoreinrichtung 11 erfasst wird. Die Photodetektoreinrichtung 11 ist vorzugsweise ein Vielkanaldetektor wie eine CCD- Kamera oder ein Photodioden- Array.

Das Signal von der Photodetektoreinrichtung 11 wird in einem ersten Speicher 12 als Intensitätssignal des Sekundärlichts 7 in Abhängigkeit von der Wellenlänge als erstes Raman- Spektralsignal in dem Speicher 12 gespeichert. Dieses abgespeicherte Spektrum ist das Spektrum für die erste Primärlichtwellenlänge λ₁. Dabei kann durch Auswahl der eingelesenen Kanäle der Vielkanalphotodetektoreinrichtung 11 zusätzlich zur optischen eine elektronische Einschränkung auf einen vorgegebenen Wellenlängenbereich vorgenommen werden.

Zum Erzeugen von monochromatischem Primärlicht 3 einer zweiten Wellenlänge λ₂ ist eine zweite Lichtquelle 13 vorgesehen. Das Primärlicht von dieser zweiten Lichtquelle 13 wird über einen ersten Strahlteiler 14 und einen zweiten Strahlteiler 15 in den Strahlengang der ersten Lichtquelle 2 eingekoppelt, so dass das Primärlicht 3 der zweiten Wellenlänge λ₂ auf identische Art in das Körpergewebe 1 eingestrahlt wird wie das Primärlicht 3 der ersten Wellenlänge λ₁.

In einem zweiten Speicher 19 wird analog zu oben die Intensität des Sekundärlichts 7 in Abhängigkeit von der Wellenlänge als zweites Raman- Spektralsignal abgespeichert, wobei gegebenenfalls auch hier eine zusätzliche elektronische Einschränkung auf einen vorgegebenen Wellenlängenbereich stattfinden kann. Dieses abgespeicherte Spektrum ist das Spektrum für die zweite Primärlichtwellenlänge λ₂.

Das Umschalten des Ausgangs der Photodetektoreinrichtung 11 auf den zweiten Speicher 19 erfolgt mit einem Schaltgatter 18, das zwischen die Photodetektoreinrichtung 11 einerseits und den ersten Speicher 12 bzw. zweiten Speicher 19 andererseits geschaltet ist. Das Gatter 18 wird durch eine Systemuhr 16 über elektrische Steuerleitungen 17 synchron zu der ersten Lichtquelle 2 und der zweiten Lichtquelle 13 angesteuert, so dass die Speicher 12 und 19 nur das "eigene" Spektrum abspeichern und es nicht zu einer Überlappung der Spektren in den Speichern 12 und 19 kommt.

In einer Komparatoreinrichtung 20 werden das erste Raman-Spektralsignal in dem ersten Speicher 12 und das zweite Raman-Spektralsignal in dem zweiten Speicher 19 miteinander verglichen, und es wird ein Vergleichssignal erzeugt, bei dem spektrale Strukturen, die durch das Körpergewebe 1 bedingt sind, im wesentlichen vollständig eliminiert sind und nur noch Merkmale des Sekundärlichtes bleiben, die auf die gesuchte Substanz im Körpergewebe bzw. in der Körperflüssigkeit zurückzuführen sind. Insbesondere ist die Komparatoreinheit 20 ein Subtrahierer, der die zwei Ramanspektren in dem Speicher 12 bzw. 19 voneinander abzieht. Ebenfalls kann jedoch auch ein Dividierer als Komparatoreinheit 20 verwendet werden, und weitere mathematische Verfahren für das Aufbereiten der Daten vor und nach dem Vergleich können ebenfalls angewendet werden.

Als mathematische Verfahren zur Aufbereitung der Daten vor und nach dem Vergleich können z.B. bekannte multivariate statistische Methoden verwendet werden, unter anderem die partielle lineare Regression, die Hauptkomponentenanalyse (principal component analysis PCA) und Hauptkomponentenregression (principal component regression PCR). Dabei wird eine Anzahl von gemessenen Raman- Spektralsignalen, die man für variable Glukosekonzentrationen erhält, mit einer multivariaten statistischen Methode analysiert. Im Unterschied zu der einfachen Differenzbildung zweier Signale erlauben diese statistischen Methoden eine besonders gute Erkennung des Glukosespektrums (pattern recognition) gegenüber dem Untergrund und damit eine genauere Bestimmung der Glukosekonzentration. Bei der praktischen Anwendung am Patienten wird eine möglichst große Anzahl von RamanSpektren zu verschiedenen Zeiten aufgenommen und abgespeichert. Jedem abgespeicherten Raman- Spektrum entspricht eine bestimmte Glukosekonzentration im Blut als Variable. Je mehr RamanSpektren als Funktion der Glukosekonzentration für die multivariate statistische Analyse zur Verfügung stehen, umso besser und genauer lässt sich die Glukosekonzentration aus der statistischen Regressionsanalyse bestimmen.

Im einzelnen wird bei dem Verfahren zum nicht- invasiven Nachweis einer Substanz in einer Körperflüssigkeit in einem Körpergewebe 1 mit Raman- Spektroskopie monochromatisches Primärlicht 3 einer ersten Wellenlänge λ₁ in das Körpergewebe 1 bei einem ersten Parameterwert eingestrahlt. Anschließend wird das Sekundärlicht 7, das von dem Körpergewebe zurückgestreut wird, erfasst und die Intensität des Sekundärlichts in Abhängigkeit von der Wellenlänge als erstes Raman- Spektralsignal bei der ersten Wellenlänge λ₁ und bei dem ersten Parameterwert in einem ersten Speicher 12 abgespeichert. Diese beiden Schritte des Einstrahlens der Primärwellenlänge und des Erfassens und Abspeicherns des Sekundärlichtes bei weiteren Primärwellenlängen und weiteren Parameterwerten wiederholt, wobei die Intensität des Sekundärlichtes 7 bei jeder weiteren Primärwellenlänge λᵢ, λⱼ, λₖ etc. und bei jedem weiteren Parameterwert in einem jeweils weiteren Speicher abgespeichert wird. Die Raman- Spektralsignale werden schließlich mit einer multivariaten statistischen Methode verglichen, um wenigstens eine Abfragewellenlänge λ₃ zu bestimmen, bei der sich die Intensität analog zu der Änderung der Parameterwerten verhält, sich also die Intensität analoge zu den Parameterwerten ändert. Die Intensität bei der Abfragewellenlänge λ₃ wird in den mehreren Raman- Spektralsignalen bestimmt und als Funktion der Parameterwerte ausgegeben.

Vorzugsweise ist der Parameter der Blutzuckerspiegel des Patienten, d.h. man misst z.B. über einen längeren Zeitraum, über den sich der Blutzuckerspiegel ändert (z.B. 20 Spektren über einen Tag), mehrere Spektren und vergleicht sie mit multivariaten statistischen Methoden.

Die Merkmale im Vergleichssignal, die auf die gesuchte Substanz im Körpergewebe bzw. in der Körperflüssigkeit zurückzuführen sind, werden in einer Diskriminatoreinheit 21 analysiert. Insbesondere wird die Intensität des Vergleichssignals bei wenigstens einer Abfragewellenlänge λ₃ bestimmt, bei der ein Signal der gesuchten Substanz erwartet wird, wobei die Intensität der Konzentration der Substanz in der Körperflüssigkeit entspricht.

Das Ergebnis der Messung wird über eine Ausgabeeinrichtung 22 dargestellt, wobei diese Ausgabeeinrichtung 22 ein einfacher Bildschirm, ein Drucker oder ein PC mit allen entsprechenden und allgemein bekannten Ausgabemöglichkeiten ist.

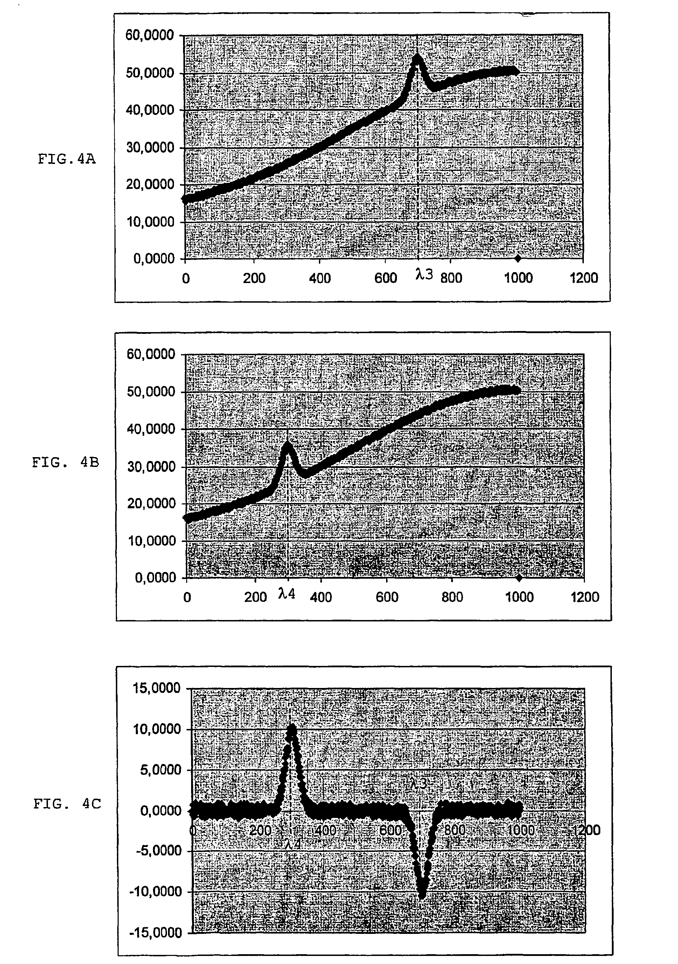

Die Raman- Spektren in dem ersten Speicher 12, dem zweiten Speicher 19 und in der Diskriminatoreinheit 21 sind als Simulation in Fig. 4A bis C dargestellt. In den Fig. 4A bis C ist die Wellenlänge des spektral zerlegten Sekundärlichts 10 als Kanalnummer der Photodetektoreinrichtung 11 auf der Abszisse aufgetragen. Die Höhe des Signals von der Photodetektoreinrichtung 11, die der Lichtintensität entspricht, ist auf der Ordinate in beliebigen Einheiten dargestellt. Im wesentlichen zeigt das Spektrum in Fig. 4A einen Intensitätsverlauf, der zu größeren Kanalnummern (Wellenlängen), also nach rechts im Spektrum ansteigt. Diesem Verlauf ist eine Raman- Linie bei einer Wellenlänge λ₃ überlagert (hier ist nur eine Linie, z.B. Stokes- Linie, dargestellt, tatsächlich sind aber zwei Linien zu erwarten, nämlich außer der Stokes- auch eine Anti- Stokes- Linie, die allerdings schwächer ist).

Im wesentlichen das gleiche Spektrum ergibt sich bei einer zweiten Wellenlänge des Primärlichts, das in Fig. 4B gezeigt ist, wobei nach Erkenntnis des Erfinders der im wesentlichen unstrukturierte Untergrund (Anstieg nach rechts) im wesentlichen unverändert ist gegenüber dem Spektrum in Fig. 4A, das für die erste Wellenlänge des Primärlichts aufgenommen wurde. Auch in Fig. 4B ist eine Raman- Linie dem breiten Anstieg überlagert, die bei der Wellenlänge λ₄ liegt. Die Energiedifferenz zwischen der Raman- Linie bei der Wellenlänge λ₃ in dem ersten Spektrum und bei der Wellenlänge λ₄ in dem zweiten Spektrum entspricht der Energiedifferenz zwischen der ersten und der zweiten Wellenlänge des Primärlichtes λ₁ bzw. λ₂ von der ersten Lichtquelle 2 bzw. zweiten Lichtquelle 13.

In Fig. 4C ist das Differenzspektrum der beiden Spektren in Fig. 4A und 4B gezeigt, bei dem der Untergrund, der auf das umliegende Körpergewebe zurückzuführen ist, eliminiert ist. Damit ist die Intensität der beiden Raman- Linien bei den Wellenlängen λ₃ bzw. λ₄ bis auf das Vorzeichen eindeutig bestimmbar und ergibt ein Maß für die Konzentration der gesuchten Substanz. (Zusätzlich erscheinen in den Differenzspektren auch entsprechende hier nicht dargestellte Anti- Stokes- Linien, die gegebenenfalls auch oder zusätzlich zur Bestimmung der Konzentration herangezogen werden können.)

Bei der Ausführungsform nach Fig. 1 ist weiterhin eine dritte Lichtquelle 24 vorgesehen, deren Licht über einen Strahlteiler 23 in den Strahlengang zwischen der ersten Lichtquelle 2 und der Abbildungs- /Auffangoptik 6 eingekoppelt wird. (Der Strahlengang in dieser und in den folgenden Figuren ist nur symbolisch zu verstehen. Dem Fachmann ist klar, dass der genaue Aufbau der Optik von den jeweiligen Transmissions- und Reflexionseigenschaften der verwendeten optischen Elemente abhängt. So kann der Strahlteiler 23 auch zwischen dem Photodetektor 9 and dem Auskoppelstrahlteiler 5 angeordnet werden. Gegebenenfalls kann die dritte Lichtquelle 24 unabhängig von der ersten Lichtquelle 2 und der zweiten Lichtquelle 13 in das Körpergewebe 1 eingestrahlt werden.) Auch für den Strahlteiler 23 kann wie für den Auskopplungsstrahlteiler 5 ein Notch- Filter eingesetzt werden, das Licht mit der Wellenlänge der dritten Lichtquelle reflektiert und alle anderen Wellenlängen durchlässt. Die dritte Lichtquelle 24 wird kurz vor der ersten bzw. zweiten Lichtquelle 2 und 13 aktiviert. Damit lässt sich das gesuchte Molekül "präparieren", z.B. lassen sich höhere Schwingungszustände in dem Molekül bevölkern bzw. untere Schwingungszustände des Moleküls depopulieren. Damit lassen sich Intensitätsunterschiede im spektralen Signal induzieren und die Selektivität bei der Anregung der gesuchten Substanz durch das Primärlicht wird angehoben. Insbesondere lässt sich dies Verfahren auf Anti- Stokes- Übergänge anwenden, die u.U. nur so ermöglicht werden und damit eine besonders empfindliche Messsonde darstellen. Die von der dritten Lichtquelle 24 erzeugte Wellenlänge λ_{Anr} liegt daher zwischen 1,2µm und 3µm. In diesem Spektralbereich ist das Köpergewebe im wesentlichen transparent. Auch in diesem Bereich wird als Lichtquelle 24 vorzugsweise ein Laser verwendet.

Aus Gründen der Relaxation der durch die dritte Lichtquelle 24 angeregten Zustände in dem gesuchten Molekül erzeugen die erste, zweite und dritte Lichtquelle 2, 13 und 24 vorzugsweise Pulse mit einer Pulslänge im Pikosekundenbereich. Dies lässt sich besonders gut mit Lasern als Lichtquelle erreichen.

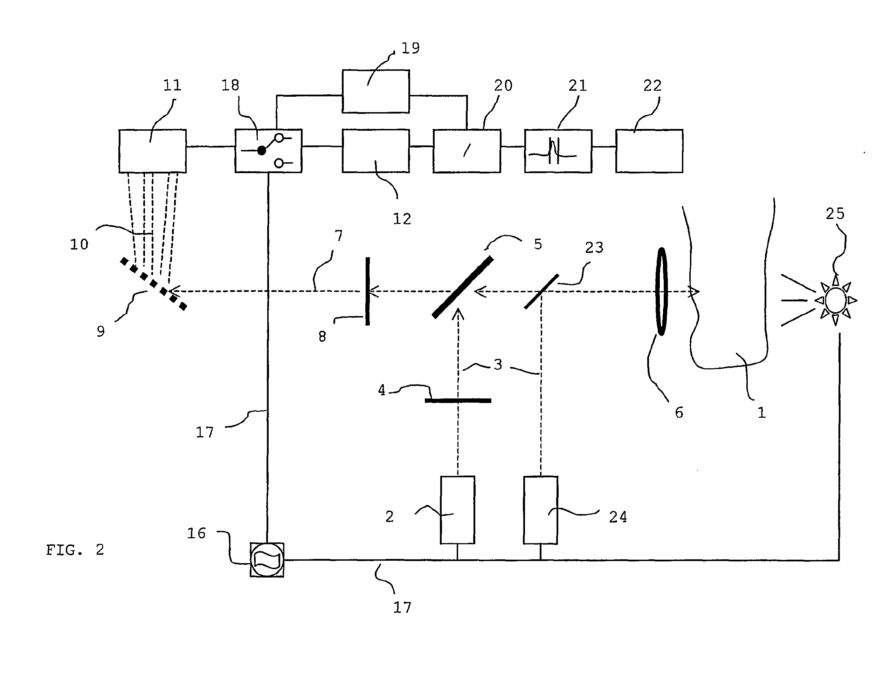

In Fig. 2 ist eine alternative Vorrichtung zum nicht- invasiven Bestimmen einer Konzentration einer Substanz in einer Körperflüssigkeit in einem Körpergewebe 1 mit Raman- Spektroskopie dargestellt, wobei gleiche Bestandteile wie bei der Vorrichtung nach Fig. 1 mit denselben Bezugszeichen bezeichnet sind und nicht noch einmal erläutert werden. Diese zweite Vorrichtung basiert auf dem Nachweis der Temperaturabhängigkeit des Messsignals. Der Aufbau dieser Vorrichtung ist im wesentlichen identisch zu dem der Vorrichtung nach Fig. 1, lediglich die zweite Lichtquelle fehlt in Fig. 2. Es ist also eine Lichtquelle 2 zum Einstrahlen von monochromatischem Primärlicht 3 einer vorgegebenen Wellenlänge λ in das Körpergewebe 1 vorgesehen. Statt einer zweiten Lichtquelle ist eine Heizeinrichtung 25 zum Einstellen einer ersten Temperatur und einer zweiten Temperatur in dem Körpergewebe 1 vorgesehen, wobei die Heizeinrichtung 25 vorzugsweise ein Infrarotstrahler ist, der rechts in Fig. 2 durch eine sternförmige Struktur angedeutet ist.

Die optischen Elemente 4 bis 6 und 8 sowie die Photodetektoreinrichtung 11 mit dispergierendem Element 9 zum Erfassen von Sekundärlicht 7, das von dem Körpergewebe 1 zurückgestreut wird, der erste Speicher 12 zum Abspeichern des ersten Raman-Spektralsignals und der zweite Speicher 19 zum Abspeichern des zweiten Raman- Spektralsignals sowie die Komparatoreinrichtung 20 und die Diskriminatoreinrichtung 21 sind identisch zu den entsprechenden Elementen in Fig. 1.

Die Messung wird zuerst bei einer ersten Temperatur T₁ des Körpergewebes 1 durchgeführt, und das entsprechende Spektrum wird in dem ersten Speicher 12 abgespeichert. Sodann wird die Messung mit derselben Primärlichtwellenlänge λ bei einer zweiten Temperatur T₂ wiederholt, und das zweite Spektrum wird in dem zweiten Speicher 19 abgespeichert. Das Vergleichen und Auswerten der Spektren erfolgt in derselben Weise wie oben bei der Vorrichtung nach Fig. 1.

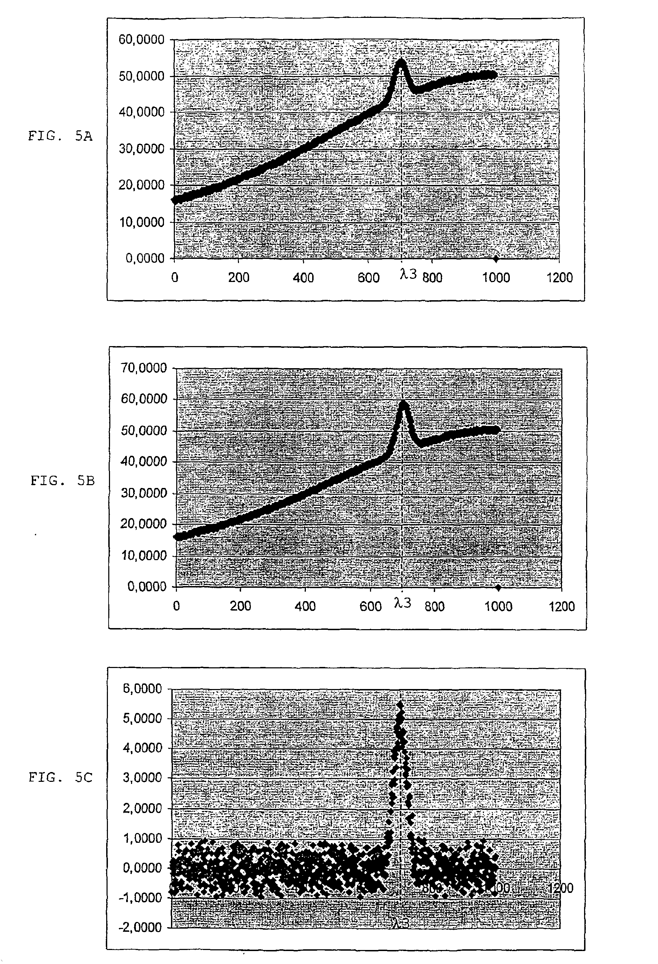

Die entsprechenden Spektren in dem ersten Speicher 12, dem zweiten Speicher 19 und in der Diskriminatoreinheit 21 sind in Fig. 5A bis C dargestellt. Hier liegen die beiden Raman- Linien beide bei der gleichen Wellenlänge λ₃, da die Anregungswellenlänge λ sich nicht verändert hat, die gestreute Linie hat also in beiden Messdurchgängen dieselbe Wellenlänge. Im übrigen weisen die Spektren in Fig. 5A und 5B ebenfalls einen breiten Anstieg auf, der nach Beobachtungen des Erfinders auf das Körpergewebe zurückzuführen ist und also nicht durch die gesuchte Substanz bedingt ist.

Das Differenzspektrum in Fig. 5C weist im Unterschied zu dem Spektrum in Fig. 4C nur eine Linie auf, nämlich die bei der Wellenlänge λ₃. Auch bei dieser Linie ist die Intensität ein Maß für die Konzentration der Substanz in der Körperflüssigkeit.

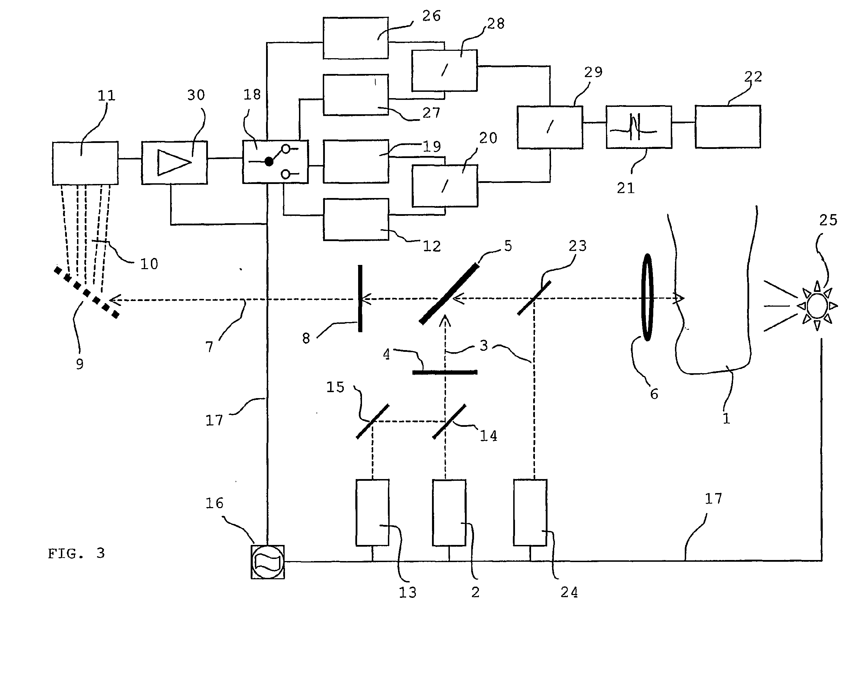

Bei manchen Substanzen ist das Raman- Signal sehr schwach, und es müssen zur Überwindung der extremen Schwierigkeiten bei dem Herausfiltern des Raman- Signals der gesuchten Substanz aus dem Hindergrundsignal des Körpergewebes gleichzeitig mehrere verschiedene Parameterverändert werden, um ein ausreichendes Signal zu erhalten. Es mag sogar so sein, dass eine Intensitätsmessung nicht mehr möglich ist, sondern nur noch ein qualitativer Nachweis der Substanz geführt werden kann. In Fig. 3 ist eine Vorrichtung zum nicht- invasiven Bestimmen einer Konzentration bzw. zum Nachweis einer Substanz in einer Körperflüssigkeit in einem Körpergewebe 1 mit Raman- Spektroskopie dargestellt, wobei gleiche Bestandteile wie bei der Vorrichtung nach Fig. 1 oder Fig. 2 mit denselben Bezugszeichen bezeichnet sind und nicht noch einmal erläutert werden.

Die Vorrichtung nach Fig. 3 umfasst eine erste Lichtquelle 2 und eine zweite Lichtquelle 13 zum Einstrahlen von monochromatischem Primärlicht 3 einer ersten Wellenlänge λ₁ bzw. einer zweiten Wellenlänge λ₂ in das Körpergewebe 1. Ferner umfasst die Vorrichtung eine Heizeinrichtung 25 zum Einstellen einer ersten Temperatur und einer zweiten Temperatur in dem Körpergewebe 1. Mit einer Photodetektoreinrichtung 11 wird das spektral zerlegte Sekundärlicht 7 aufgenommen, und in einem ersten Speicher 19 wird ein erstes Raman- Spektralsignal für die erste Primärlichtwellenlänge λ₁ und bei der ersten Temperatur T₁ aufgenommen, in einem zweiten Speicher 19 wird ein zweites Raman-Spektralsignal für die zweite Primarlichtwellenlänge λ₂ und bei der ersten Temperatur T₁ aufgenommen, in einem dritten Speicher 26 wird ein drittes Raman- Spektralsignal für die erste Primärlichtwellenlänge λ₁ und bei der zweiten Temperatur T₂ aufgenommen, und in einem vierten Speicher 27 wird ein viertes Raman-Spektralsignal für die zweite Primärlichtwellenlänge λ₂ und bei der zweiten Temperatur T₂ aufgenommen.

In einer ersten Komparatoreinrichtung 20 werden das erste Raman- Spektralsignal in dem ersten Speicher 12 und das zweite Raman- Spektralsignal in dem zweiten Speicher 19 miteinander verglichen und ein erstes Vergleichssignal erzeugt, bei dem spektrale Strukturen, die durch das Körpergewebe bedingt sind, im wesentlichen vollständig eliminiert sind.

Analog werden in einer zweiten Komparatoreinrichtung 28 das dritte Raman- Spektralsignal in dem dritten Speicher 26 und das vierte Raman- Spektralsignal in dem vierten Speicher 27 miteinander verglichen und ein zweites Vergleichssignal erzeugt, bei dem spektrale Strukturen, die durch das Körpergewebe bedingt sind, im wesentlichen vollständig eliminiert sind.

In einer dritten Komparatoreinrichtung 29 werden das erste Vergleichssignal von der ersten Komparatoreinrichtung 20 und das zweite Vergleichssignal von der zweiten Komparatoreinrichtung 28 miteinander verglichen und ein drittes Vergleichssignal in Abhängigkeit von dem Vergleichsergebnis erzeugt.

In einer Diskriminatoreinrichtung 21 wird die Intensität des dritten Vergleichssignals bei wenigstens einer Abfragewellenlänge λ₃ bestimmt, und in einer Ausgabeeinheit 22 wird ein Identifikationssignal ausgegeben, das anzeigt, ob bei der wenigstens einen Abfragewellenlänge λ₃ eine temperaturabhängige Signalveränderung vorliegt. Ist der temperaturabhängige Unterschied zwischen dem ersten Vergleichssignal und dem zweiten Vergleichssignal groß genug, so kann daraus wiederum eine Angabe über die Konzentration des gesuchten Substanz in der Körperflüssigkeit abgeleitet werde, wobei zur Berechnung auf die bekannte Temperaturdifferenz zwischen den Messungen zurückgegriffen wird. Damit wird erfindungsgemäß eine sehr empfindliche Mehrfachdifferentialvorrichtung zum Nachweis von Substanzen geschaffen.

Da die Messungen mit der Vorrichtung nach Fig. 3 u.U. beeinträchtigt sind durch statistisches Rauschen, wird die erste Lichtquelle 2 und die zweite Lichtquelle 13 gepulst betrieben. Synchron zur Ansteuerung der Lichtquellen 2 und 13 durch die Systemuhr 16 werden die Photodetektoreinrichtung 11 und der erste Speicher 12, der zweite Speicher 19, der dritte Speicher 26 und der vierte Speicher 27 mit einer vorgegebenen Frequenz betrieben. So werden beispielsweise 100 Messungen bei der ersten Primärlichtwellenlänge λ₁ und der ersten Temperatur T₁ durchgeführt, 100 Messungen bei der ersten Primärlichtwellenlänge λ₁ und der zweiten Temperatur T₂, etc.. Als Zwischenspeicher ist für diese Art Messungen ein lock- in- Verstärker 30 vorgesehen, der ebenfalls durch die Systemuhr 16 angesteuert und synchronisiert wird.

Die Frequenz, mit der Messsequenzen durchgefahren werden, liegen vorzugsweise im Bereich von einigen Hertz bis zu einigen Kilohertz, so dass die Aufnahme von 100 Messungen für zwei Primärlichtwellenlängen bei einer Frequenz der Messsequenz von einigen Hertz bis Kilohertz innerhalb von wenigen Minuten abgeschlossen ist.

Die Einstellung der Temperatur in dem Körpergewebe dauert naturgemäß etwas länger, so dass die Frequenz, mit der die Heizvorrichtung 25 zum Einstellen der ersten und zweiten Temperatur des Körpergewebes angesteuert wird, vorzugsweise die Herzfrequenz ist. Dazu ist bei der Vorrichtung nach Fig. 2 oder 3 eine (nicht dargestellte) Aufnahmevorrichtung zum Erfassen der Herzfrequenz vorgesehen.

Für den Nachweis von Glukose in Körperflüssigkeiten in einem Körpergewebe 1 erzeugen die Lichtquellen eine Wellenlänge λ₁ bzw. λ₂ des Primärlichtes 3 zwischen 750nm und 850nm. In diesem Wellenlängenbereich ist das Körpergewebe 1 im wesentlichen transparent.

Mit den obigen Vorrichtungen lassen sich die im folgenden beschriebenen Verfahren zum Nachweis bzw. zur Konzentrationsbestimmung von Substanzen durchführen.

Ein erfindungsgemäßes Verfahren zum nicht- invasiven Bestimmen einer Konzentration einer Substanz in einer Körperflüssigkeit in einem Körpergewebe 1 mit Raman- Spektroskopie umfasst als ersten Schritt das Einstrahlen von monochromatischem Primärlicht 3 einer ersten Wellenlänge λ₁ in das Körpergewebe 1. Das Sekundärlicht 7, das von dem Körpergewebe zurückgestreut wird, wird erfasst, und seine Intensität wird in Abhängigkeit von der Wellenlänge als erstes Raman- Spektralsignal in einem ersten Speicher 12 abgespeichert.

Wie oben bereits erläutert und in Fig. 4A, 4B, 5A und 5B ersichtlich, umfasst ein Raman- Spektrum bereits Information über die gesuchte Substanz. Wegen der sehr großen Komplexität der Zusammensetzung menschlichen Gewebes und Bluts ist es jedoch notwendig, das Hintergrundsignal des Körpergewebes zu eliminieren. Das erfindungsgemäße Verfahren beruht auf einem Detektieren eines ersten Intensitätssignals bei einem der nachzuweisenden Substanz für die erste Primärlichtwellenlänge (λ₁) entsprechenden Wellenlängenspektrum und einem anschließenden Detektieren eines zweiten Intensitätssignals bei einem entsprechend der zweiten Primärlichtwellenlänge (λ₂) gegenüber der ersten Primärlichtwellenlänge verschobenen Wellenlängenspektrum. Im folgenden wird der Einfachheit halber Glukose als nachzuweisende Substanz angenommen; sinngemäß sind die erfindungsgemäßen Verfahren und Vorrichtungen auch auf andere Substanzen anzuwenden. Bei der Glukose handelt es sich um ein gegenüber den anderen im Körpergewebe und Blut vorhandenen Substanzen relativ kleines Molekül, welches im Blut oder im Körpergewebe ein scharfes Raman- Spektrum mit Linienbreiten von ca. 2,5meV (20cm⁻¹) aufweist. Dies entspricht bei einer Primärlichtwellenlänge von ca. 800nm einer Linienbreite von ca. 1,3nm, einer typischen Linienbreite von Diodenlasern in diesem Wellenlängenbereich. Das Hintergrund Raman- Spektrum des Bluts oder Körpergewebes, welches überwiegend von sehr großen Molekülen und von Wasser herrührt, ist demgegenüber spektral sehr breit. Wird also die Primärlichtwellenlänge um ein geringfügiges Vielfaches der Bandbreite eines Raman- Übergangs der Glukose verschoben, also um wenige nm, so führt dies für das Raman- Spektrum der Glukose zu einer klar ausgeprägten Verschiebung, während das Hintergrund Raman- Spektrum weitestgehend unverändert bleibt. Entsprechend dem erfindungsgemäßen Verfahren werden also zwei Raman- Spektren bei unterschiedlichen Primärwellenlängen aufgenommen. Durch Subtrahieren beider Raman- Spektren wird das Hintergrundspektrum eliminiert und man erhält das von der Glukose stammende Raman- Spektrum in diesem Differenzspektrum Spektrum in diesem Differenzspektrum als Spektrum von positiven und negativen Peaks auf einer Basislinie. Diese Basislinie sollte im Idealfall konstant sein. Wegen der zwar geringen, aber nicht vollständig vernachlässigbaren Abhängigkeit des Hintergrund Raman- Spektrums von der Primärwellenlänge ist diese Basislinie unter realen Messbedingungen nicht vollständig konstant, sondern zeigt eine sehr breite Struktur, die aber leicht von den gesuchten schmallinigen Strukturen im Spektrum unterschieden werden kann. Erfindungsgemäß ergeben sich auf dieser Basislinie für jeden Schwingungsübergang der Glukose ein gegenüber der Basislinie positiver und negativer Peak, wobei die Peakmaxima entsprechend dem Wellenlängenunterschied (Wellenzahlunterschied, Energiedifferenz) des Primärlichtes gegeneinander verschoben sind und das Intensitätssignal in der Mitte zwischen dem positiven und negativen Peak dem der Basislinie entspricht. Bei bekanntem Wellenlängenunterschied beider Primärlichtwellenlängen und bekannter Bandbreite und Peakform der Peaks des Glukose Raman- Spektrums kann also ein der Glukosekonzentration proportionales Intensitätssignal durch Datenanalyse des Differenzspektrums und Vergleich mit gemessenen und modellierten Spektren erhalten werden.

Das Verfahren umfasst daher als weiteren Schritt ein Einstrahlen von monochromatischem Primärlicht 3 einer zweiten Wellenlänge λ₂ in das Körpergewebe 1 sowie das Erfassen von Sekundärlicht 7, das von dem Körpergewebe 1 bei der zweiten Primärwellenlänge zurückgestreut wird. Die Intensität des Sekundärlichts wird in Abhängigkeit von der Wellenlänge als zweites Raman- Spektralsignal bei der zweiten Wellenlänge λ₂ in einem zweiten Speicher 19 abgespeichert. Das erste Raman- Spektralsignal in dem ersten Speicher 12 und das zweite Raman- Spektralsignal in dem zweiten Speicher 19 werden miteinander verglichen, und es wird ein Vergleichssignal erzeugt, bei dem spektrale Strukturen, die durch das Körpergewebe bedingt sind, im wesentlichen vollständig eliminiert sind. Aus der Intensität des Vergleichssignals bei wenigstens einer Abfragewellenlänge λ₃ wird die Konzentration der Substanz in der Körperflüssigkeit ermittelt.

Analog wird bei der Vorrichtung nach Fig. 2 ein Verfahren zum nicht- invasiven Bestimmen einer Konzentration einer Substanz in einer Körperflüssigkeit in einem Körpergewebe 1 mit Raman-Spektroskopie durchgeführt, das die Schritte umfasst: a) Einstrahlen von monochromatischem Primärlicht 3 einer vorgegebenen Wellenlänge λ in das Körpergewebe 1 bei einer ersten Temperatur, b) Erfassen von Sekundärlicht 7, das von dem Körpergewebe zurückgestreut wird, und Abspeichern der Intensität des Sekundärlichts in Abhängigkeit von der Wellenlänge als erstes Raman- Spektralsignal bei der ersten Temperatur T₁ in einem ersten Speicher 12, c) Einstrahlen von monochromatischem Primärlicht 3 der vorgegebenen Wellenlänge λ in das Körpergewebe 1 bei einer zweiten Temperatur, d) Erfassen von Sekundärlicht 7, das von dem Körpergewebe zurückgestreut wird, und Abspeichern der Intensität des Sekundärlichts in Abhängigkeit von der Wellenlänge als zweites Raman- Spektralsignal bei der zweiten Temperatur T₂ in einem zweiten Speicher 19, e) Vergleichen des ersten Raman- Spektralsignals in dem ersten Speicher 12 und des zweiten Raman- Spektralsignals in dem zweiten Speicher 19 miteinander und Erzeugen eines Vergleichssignals, bei dem spektrale Strukturen, die durch das Körpergewebe bedingt sind, im wesentlichen vollständig eliminiert sind, f) Bestimmen der Intensität des Vergleichssignals bei wenigstens einer Abfragewellenlänge λ₃, wobei die Intensität der Konzentration der Substanz in der Körperflüssigkeit entspricht.

Die Intensität der Raman- Streung ist proportional der Besetzung des Energieniveaus, aus dem die Streuung erfolgt; die Besetzung der Energieniveaus in Abhängigkeit von der absoluten Temperatur (in Kelvin) ist durch die Boltzmann- Verteilung gegeben. Bei einer Erhöhung der Temperatur wird die Besetzung der Moleküle in angeregten Schwingungsniveaus erhöht und entsprechend die Besetzung im Schwingungsgrundzustand erniedrigt (und umgekehrt). Das Intensitätssignal der Stokes- Raman- Streuung (auf der gegenüber der Primärlichtwellenlänge längerwelligen Seite) wird also bei einer Temperaturerhöhung des Körpergewebes verringert, während das Intensitätssignal der anti- Stokes- Raman- Streuung (auf der gegenüber der Primärlichtwellenlänge kürzerwelligen Seite) erhöht wird. Eine Temperaturänderung um 3K bewirkt eine Änderung des Raman- Signals der nachzuweisenden Substanz von 1%. Mit einer für das menschliche Körpergewebe realistisch möglichen Temperaturänderung von ca. 10K (z.B. in der Stirn) lässt sich also eine Intensitätsänderung des Raman- Signals von über 3% erreichen. Damit kann die Glukosekonzentration im Blut mit der erforderlichen Empfindlichkeit und Genauigkeit bestimmt werden.

Bei dem erfindungsgemäßen Verfahren wird die Temperaturabhängigkeit der Raman- Streuung ausgenutzt, um ein mit der Konzentration der nachzuweisenden Substanz korreliertes Signal zu erhalten. Zur Bestimmung der Konzentration der nachzuweisenden Substanz im Körpergewebe wird Primärlicht in mindestens einem Wellenlängenbereich eingestrahlt und das Sekundärlicht wird erfasst und spektral zerlegt. Das Eliminieren des Hintergrundsignals des Bluts und des Körpergewebes wird erfindungsgemäß dadurch erreicht, dass das Raman- Spektrum der nachzuweisenden Substanz für mindestens zwei verschiedene Temperaturen des Körpergewebes aufgenommen wird. Das Intensitätssignal des Raman-Spektrums der nachzuweisenden Substanz hängt von der Temperatur ab und ist entsprechend der unterschiedlichen Temperatur des Körpergewebes unterschiedlich. Demgegenüber ist das Raman-Spektrum des Körpergewebes weitestgehend unabhängig von seiner Temperatur. Durch Subtrahieren beider Raman- Spektren sowie durch Datenanalyse des Differenzspektrums und Vergleich mit gemessenen und modellierten Spektren lässt sich also das Hintergrundsignal des Körpergewebes eliminieren und ein Signal gewinnen, welches proportional zu der Konzentration der nachzuweisenden Substanz ist.

Für den qualitativen Nachweis einer gesuchten Substanz bzw. zur Erhöhung der Empfindlichkeit und Genauigkeit umfasst das erfindungsgemäße Verfahren zum nicht- invasiven Bestimmen einer Konzentration bzw. Nachweis einer Substanz in einer Körperflüssigkeit in einem Körpergewebe 1 mit Raman- Spektroskopie nach Fig. 3 die Schritte a) Einstrahlen von monochromatischem Primärlicht 3 einer ersten Wellenlänge λ₁ in das Körpergewebe 1 bei einer ersten Temperatur, b) Erfassen von Sekundärlicht 7, das von dem Körpergewebe zurückgestreut wird, und Abspeichern der Intensität des Sekundärlichts in Abhängigkeit von der Wellenlänge als erstes Raman- Spektralsignal bei der ersten Wellenlänge λ₁ und bei der ersten Temperatur T₁ in einem ersten Speicher 12, c) Einstrahlen von monochromatischem Primärlicht 3 einer zweiten Wellenlänge λ₂ in das Körpergewebe 1 bei einer ersten Temperatur, d) Erfassen von Sekundärlicht 7, das von dem Körpergewebe zurückgestreut wird, und Abspeichern der Intensität des Sekundärlichts in Abhängigkeit von der Wellenlänge als zweites Raman- Spektralsignal bei der zweiten Wellenlänge λ₂ und bei der ersten Temperatur T₁ in einem zweiten Speicher 19, e) Vergleichen des ersten Raman- Spektralsignals in dem ersten Speicher 12 und des zweiten Raman- Spektralsignals in dem zweiten Speicher 19 miteinander und Erzeugen eines ersten Vergleichssignals, bei dem spektrale Strukturen, die durch das Körpergewebe bedingt sind, im wesentlichen vollständig eliminiert sind, f) Bestimmen der Intensität des ersten Vergleichssignals bei wenigstens einer Abfragewellenlänge λ₃, g) Wiederholen der Schritte a) bis f) bei einer zweiten Temperatur und Erzeugen eines zweiten Vergleichssignals bei der wenigstens einen Abfragewellenlänge λ₃, wobei die Intensität des Sekundärlichtes 7 bei der ersten und zweiten Wellenlänge λ₁, λ₂ in einem dritten Speicher 26 bzw. einem vierten Speicher 28 abgespeichert wird und ein zweites Vergleichssignal erzeugt wird, h) Vergleichen des ersten und des zweiten Vergleichssignals und Erzeugen eines Identifikationssignals in Abhängigkeit von dem Vergleichsergebnis, das anzeigt, ob bei der wenigstens einen Abfragewellenlänge λ₃ eine temperaturabhängige Signalveränderung vorliegt. Das Verfahren dieser Art stellt ein sehr empfindliches Mehrfachdifferentialverfahren zum Nachweis von Substanzen dar. Insbesondere kann mit der Änderung beider Parameter die Restwelligkeit (Verschiebung der Hintergrundstrukturen im Differenzspektrum) in den beiden ersten Vergleichsspektren eliminiert werden, so dass die Basislinie im Differenzspektrum bei diesem Verfahren noch weniger Strukturen aufweist als bei dem "einfachen" Vergleich. Es lässt sich so eine höhere Genauigkeit und Reproduzierbarkeit der Konzentrationsbestimmung der gesuchten Substanz erreichen. Die Temperaturänderungen zwischen T₁ und T₂ sind vorzugsweise mit dem Zyklus Systole/Diastole des Blutkreislaufs synchronisiert.

Bei dem Verfahren mit der Vorrichtung nach Fig. 3 wird das Primärlicht 3 der ersten und/oder zweiten Wellenlänge vorzugsweise mit einer vorgegebenen Frequenz eingestrahlt, und das erste und das zweite Raman- Spektralsignal wird mit einem lock-in- Verstärker 30 bei der vorgegebenen Frequenz aufgenommen und abgespeichert. Dabei liegt die vorgegebene Frequenz vorzugsweise im Bereich von einigen Hertz bis Kilohertz.

Alle Verfahren werden vorzugsweise bei Sekundärlicht 7 im Stokes- Bereich und/oder Anti- Stokes- Bereich des Raman-Spektrums durchgeführt, um die Nachweis- und Messempfindlichkeit noch weiter zu steigern. Das bedeutet, dass der erfasste Wellenlängenbereich bei Sekundärlicht zu kürzeren Wellenlängen als der Primärlichtwellenlänge λ₁, λ₂ (Anti- Stokes) bzw. zu längeren Wellenlängenbereichen (Stokes) verschoben ist.

Die Wellenlänge λ₁, λ₂ des Primärlichts 3 liegt in einem Bereich in dem das Körpergewebe im wesentlich transparent ist, also zwischen 750nm und 850nm, und der Absolutwert der Differenz zwischen der Wellenlänge λ₁, λ₂ des Primärlichts 3 und der Abfragewellenlänge λ₃ ist z.B. für Glukosemessungen vorzugsweise kleiner als 250meV (2000cm⁻¹), aber größer als 2,5meV (20cm⁻¹).

Darüber hinaus kann im wesentlichen zeitgleich mit dem Primärlicht 3 ein Anregungslicht mit einer Wellenlänge λ_{Anr} in das Körpergewebe 1 eingestrahlt werden, die von der Substanz absorbiert wird, so dass es zur Anregung von Schwingungsbanden in dem gesuchten Molekül kommt, was man im Messsignal verfolgen kann. Die Wellenlänge λ_{Anr} des Anregungslichtes liegt bei Glukose zwischen 1,2µm und 3µm.

Eine Eichung der Konzentration der nachzuweisenden Substanz in Körperflüssigkeiten oder im Körpergewebe erfordert ein konstantes, von der Konzentration der nachzuweisenden Substanz unabhängiges Bezugssignal. Dies erfolgt durch den Vergleich des der nachzuweisenden Substanz zugeordneten Messsignals mit einem bekannten konstanten Messsignal aus demselben Streuvolumen im Körpergewebe 1. Dazu eignen sich das weitestgehend konstante Untergrundsignal (Anstieg in Fig. 4A, 4B; 5A, 5B), die restliche Rayleigh Streuung, sowie das vom Wasser oder einer anderen Substanz, deren Konzentration bekannt oder konstant ist, stammende Raman- Signal. Durch Intensitätsvergleich des Substanzsignals mit dem Bezugssignal ergibt sich die Eichung der Konzentration der Substanz. Alternativ kann das Streuvolumen, aus dem das Sekundärlicht aufgenommen wird aus dem Bezugssignal bestimmt werden und aus dem Streuvolumen kann das Signal der nachzuweisenden Substanz geeicht werden und damit ihre Konzentration bestimmt werden. Mit anderen Worten, zum Eichen der Konzentrationsbestimmung der gesuchten Substanz wird die Intensität des ersten Vergleichssignals bei der wenigstens einen Abfragewellenlänge λ₃ mit einer Intensität des ersten und/oder zweiten Raman- Spektralsignals bei einer weiteren Abfragewellenlänge λ₄ verglichen. Die Eichung der Konzentrationsbestimmung der gesuchten Substanz kann z.B. als klinische Eichung (an anderem Ort und zu anderer Zeit als die eigentliche Messung) erfolgen.

Bei einer weiteren verbesserten Ausführungsform des erfindungsgemäßen Verfahrens erfolgt die Identifizierung des Glukosesignals und die Bestimmung der Glukosekonzentration durch an sich bekannte multivariate statistisch- mathematische Methoden zum Vergleichen und zur Analyse der gemessenen Raman- Spektralsignale. Die multivariaten statistisch- mathematischen Methoden sind dabei in einem Computersystem implementiert, das mit der erfindungsgemäßen Messvorrichtung verbunden ist. Bei der Anwendung am Patienten wird zu unterschiedlichen Zeitpunkten eine möglichst große Zahl von Raman- Spektren gemessen und in der Speichereinheit gespeichert. Wie dem Fachmann wohlbekannt ist, dienen diese Messungen dann zur Erstellung eines Kalibrationsmodells über eine multivariate statistische Regression (z.B. eine Hauptkomponentenregression). Die Glukosekonzentration für eine neue Messung lässt sich dann mit den aus dem Kalibrationsmodell erhaltenen Regressionskoeffizienten mit einer bestimmten Standardabweichung ermitteln. Je mehr Messdaten für die Auswertung zur Verfügung stehen, desto geringer wird die Standardabweichung für die Glukosekonzentration. Die Vorrichtung ist quasi in der Lage, durch "Lernen", also durch Datenakkumulation und Verbesserung des aus der multivariaten statistischen Analyse erhaltenen Kalibrationsmodells, zu einer Verbesserung der Genauigkeit der Glukosekonzentrationsbestimmung zu kommen.

Zusammenfassend: Die Erfindung betrifft ein Verfahren und eine Vorrichtung zum nicht- invasiven Nachweis bzw. Bestimmen der Konzentration von Substanzen in Körperflüssigkeiten mit Raman-Spektroskopie. Um einen nicht- invasiven in vivo Nachweis von Substanzen in Körperflüssigkeiten mit sehr guter reproduzierbarer Analysegenauigkeit bei einer für den Patienten akzeptablen, kurzen Messdauer zu ermöglichen, wird ein Verfahren und eine Vorrichtung zum Durchführen einer Raman- Spektroskopie bei einer Körperflüssigkeit in einem Körpergewebe 1 vorgeschlagen, wobei mindestens zwei Raman- Spektren unter unterschiedlichen physikalischen Bedingungen aufgenommen werden und miteinander verglichen werden. Das Vergleichsergebnis ist ein Nachweis der gesuchten Substanz bzw. ein Maß für die Konzentration der gesuchten Substanz.

Weitere Ausführungsformen, die über die oben beschriebenen hinausgehen, aber auf dem Prinzip der Erfindung aufbauen, lassen sich für den Fachmann leicht denken. So ist es selbstverständlich möglich, als erste und zweite Lichtquelle 2 und 13 eine einzige Laservorrichtung, z.B. eine Laserdiode zu verwenden, die durch Änderung eines elektrischen Parameters mit hoher Wiederholungsrate verstimmbar ist, so dass sich eine erste Wellenlänge λ₁ und eine zweite (nicht zu weit von der ersten abweichende) Wellenlänge λ₂ mit derselben Lichtquelle erzeugen lässt.

Des weiteren lassen sich die Strahlengänge 3 und 7 besonders gut an die Untersuchungsumgebung und -verhältnisse anpassen, wenn das Licht jeweils in Lichtwellenleiter oder Lichtwellenleiterbündel eingekoppelt wird bzw. von diesen aufgefangen wird.

Darüber hinaus wird in der obigen Beschreibung unter dem Begriff "Heizeinrichtung" selbstverständlich auch eine Einrichtung für wechselndes Heizen und Kühlen verstanden. Bei einer Einrichtung, die sowohl zum Heizen als auch zum Kühlen geeignet ist, lässt sich so ohne Schädigung des Körpergewebes die nutzbare Temperaturdifferenz zwischen T₁ und T₂ vergrößern, so dass sich ein besseres Signalrauschverhältnis ergibt.

Die Spektren in dem ersten, zweiten, dritten und vierten Speicher werden jeweils über einen vorgegebenen Wellenlängenbereich abgespeichert. Bei allen Verfahren und Vorrichtungen können zur Verbesserung der Messstatistik mehrere Messsequenzen durchgeführt werden, u.a. mit lock- in- Verstärkung.

### Bezugszeichen

- 1: Körpergewebe
- 2: erste Lichtquelle
- 3: Primärlicht
- 4: erstes optisches Filter
- 5: Auskopplungsstrahlteiler
- 6: Abbildungs-/Auffangoptik
- 7: Sekundärlicht
- 8: zweites optisches Filter
- 9: dispergierendes Element, Gitter
- 10: spektral zerlegtes Sekundärlicht
- 11: Photodetektoreinrichtung, CCD-Kamera
- 12: erster Speicher
- 13: zweite Lichtquelle
- 14: erster Strahlteiler
- 15: zweiter Strahlteiler bzw. Spiegel
- 16: Systemuhr
- 17: elektrische Steuerleitung
- 18: Schaltgatter
- 19: zweiter Speicher
- 20: erste Komparatoreinrichtung
- 21: Diskriminatoreinheit
- 22: Ausgabeeinrichtung
- 23: dritter Strahlteiler bzw. Spiegel
- 24: dritte Lichtquelle
- 25: Heizeinrichtung
- 26: dritter Speicher
- 27: vierter Speicher
- 28: zweite Komparatoreinrichtung
- 29: dritte Komparatoreinrichtung
- 30: Lock-in-Verstärker

## Patentansprüche

1. Verfahren zum nicht-invasiven Bestimmen einer Konzentration einer Substanz in einer Körperflüssigkeit in einem Körpergewebe (1) mit Raman-Spektroskopie, das die Schritte umfasst:
a) Einstrahlen von monochromatischem Primärlicht (3) einer ersten Wellenlänge (λ₁) in das Körpergewebe (1),
b) Erfassen von Sekundärlicht (7), das von dem Körpergewebe zurückgestreut wird, und Abspeichern der Intensität des Sekundärlichts in Abhängigkeit von der Wellenlänge als erstes Raman-Spektralsignal bei der ersten Wellenlänge (λ₁) in einem ersten Speicher (12),
c) Einstrahlen von monochromatischem Primärlicht (3) einer zweiten Wellenlänge (λ₂) in das Körpergewebe (1),
d) Erfassen von Sekundärlicht (7), das von dem Körpergewebe zurückgestreut wird, und Abspeichern der Intensität des Sekundärlichts in Abhängigkeit von der Wellenlänge als zweites Raman-Spektralsignal bei der zweiten Wellenlänge (λ₂) in einem zweiten Speicher (19),
e) Vergleichen des ersten Raman-Spektralsignals in dem ersten Speicher (12) und des zweiten Raman-Spektralsignals in dem zweiten Speicher (19) miteinander und Erzeugen eines Vergleichssignals, bei dem spektrale Strukturen, die durch das Körpergewebe bedingt sind, im wesentlichen vollständig eliminiert sind,
f) Bestimmen einer Intensität des Vergleichssignals bei wenigstens einer Abfragewellenlänge (λ₃), wobei die Intensität der Konzentration der Substanz in der Körperflüssigkeit entspricht,
wobei das Primärlicht (3) der ersten und/oder zweiten Wellenlänge mit einer vorgegebenen Frequenz eingestrahlt wird und das erste und das zweite Raman-Spektralsignal mit der vorgegebenen Frequenz aufgenommen wird.

2. Verfahren zum nicht-invasiven Bestimmen einer Konzentration einer Substanz in einer Körperflüssigkeit in einem Körpergewebe (1) mit Raman-Spektroskopie, das die Schritte umfasst:
a) Einstrahlen von monochromatischem Primärlicht (3) einer vorgegebenen Wellenlänge (λ) in das Körpergewebe (1) bei einer ersten Temperatur des Körpergewebes,
b) Erfassen von Sekundärlicht (7), das von dem Körpergewebe zurückgestreut wird, und Abspeichern der Intensität des Sekundärlichts in Abhängigkeit von der Wellenlänge als erstes Raman-Spektralsignal bei der ersten Temperatur in einem ersten Speicher (12),
c) Einstrahlen von monochromatischem Primärlicht (3) der vorgegebenen Wellenlänge (λ) in das Körpergewebe (1) bei einer zweiten Temperatur des Körpergewebes,
d) Erfassen von Sekundärlicht (7), das von dem Körpergewebe zurückgestreut wird, und Abspeichern der Intensität des Sekundärlichts in Abhängigkeit von der Wellenlänge als zweites Raman-Spektralsignal bei der zweiten Temperatur in einem zweiten Speicher (19),
e) Vergleichen des ersten Raman-Spektralsignals in dem ersten Speicher (12) und des zweiten Raman-Spektralsignals in dem zweiten Speicher (19) miteinander und Erzeugen eines Vergleichssignals, bei dem spektrale Strukturen, die durch das Körpergewebe bedingt sind, im wesentlichen vollständig eliminiert sind,
f) Bestimmen einer Intensität des Vergleichssignals bei wenigstens einer Abfragewellenlänge (λ₃), wobei die Intensität der Konzentration der Substanz in der Körperflüssigkeit entspricht.

3. Verfahren zum nicht-invasiven Nachweis einer Substanz in einer Körperflüssigkeit in einem Körpergewebe (1) mit Raman-Spektroskopie, das die Schritte umfasst:
a) Einstrahlen von monochromatischem Primärlicht (3) einer ersten Wellenlänge (λ₁) in das Körpergewebe (1) bei einer ersten Temperatur,
b) Erfassen von Sekundärlicht (7), das von dem Körpergewebe zurückgestreut wird, und Abspeichern der Intensität des Sekundärlichts in Abhängigkeit von der Wellenlänge als erstes Raman-Spektralsignal bei der ersten Wellenlänge (λ₁) und bei der ersten Temperatur in einem ersten Speicher (12),
c) Einstrahlen von monochromatischem Primärlicht (3) einer zweiten Wellenlänge (λ₂) in das Körpergewebe (1) bei einer ersten Temperatur,
d) Erfassen von Sekundärlicht (7), das von dem Körpergewebe zurückgestreut wird, und Abspeichern der Intensität des Sekundärlichts in Abhängigkeit von der Wellenlänge als zweites Raman-Spektralsignal bei der zweiten Wellenlänge (λ₂) und bei der ersten Temperatur in einem zweiten Speicher (19),
e) Vergleichen des ersten Raman-Spektralsignals in dem ersten Speicher (12) und des zweiten Raman-Spektralsignals in dem zweiten Speicher (19) miteinander und Erzeugen eines ersten Vergleichssignals, bei dem spektrale Strukturen, die durch das Körpergewebe bedingt sind, im wesentlichen vollständig eliminiert sind,
f) Bestimmen einer Intensität des ersten Vergleichssignals bei wenigstens einer Abfragewellenlänge (λ₃),
g) Wiederholen der Schritte a) bis f) bei einer zweiten Temperatur und Erzeugen eines zweiten Vergleichssignals bei der wenigstens einen Abfragewellenlänge (λ₃), wobei die Intensität des Sekundärlichtes (7) bei der ersten und der zweiten Wellenlänge (λ₁, λ₂) in einem dritten Speicher (26) bzw. einem vierten Speicher (28) abgespeichert wird und ein zweites Vergleichssignal erzeugt wird,
h) Vergleichen des ersten und des zweiten Vergleichssignals und Erzeugen eines Identifikationssignals in Abhängigkeit von dem Vergleichsergebnis bei der wenigstens einen Abfragewellenlänge (λ₃).

4. Verfahren nach Anspruch 3, **dadurch gekennzeichnet, dass** das Primärlicht (3) der ersten und/oder zweiten Wellenlänge mit einer vorgegebenen Frequenz eingestrahlt wird und das erste und das zweite Raman-Spektralsignal mit der vorgegebenen Frequenz aufgenommen wird.

5. Verfahren nach Anspruch 4, **dadurch gekennzeichnet, dass** die vorgegebene Frequenz im Bereich von einigen kHz liegt.

6. Verfahren nach Anspruch 2 oder 3, **dadurch gekennzeichnet, dass** die erste und die zweite Temperatur des Körpergewebes mit einer vorgegebenen Frequenz eingestellt wird.

7. Verfahren nach Anspruch 6, **dadurch gekennzeichnet, dass** die vorgegebene Frequenz die Herzfrequenz ist.

8. Verfahren nach einem der vorangehenden Ansprüche, **dadurch gekennzeichnet, dass** das Primärlicht (3) mit der ersten und/oder zweiten Wellenlänge eine Pulslänge im Pikosekundenbereich aufweist.

9. Verfahren nach Anspruch 8, **dadurch gekennzeichnet, dass** das Primärlicht durch einen Laser erzeugt wird.

10. Verfahren nach einem der vorangehenden Ansprüche, **dadurch gekennzeichnet, dass** das Sekundärlicht (7) im Stokes-Bereich und/oder Anti-Stokes-Bereich des Raman-Spektrums erfasst wird.

11. Verfahren nach einem der vorangehenden Ansprüche, **dadurch gekennzeichnet, dass** die Wellenlänge (λ₁, λ₂) des Primärlichts (3) zwischen 750nm und 850nm liegt.

12. Verfahren nach einem der vorangehenden Ansprüche, **dadurch gekennzeichnet, dass** der Absolutwert der Differenz zwischen der Wellenlänge (λ₁, λ₂) des Primärlichts (3) und der Abfragewellenlänge (λ₃) kleiner als 2000cm⁻¹ ist.

13. Verfahren nach einem der vorangehenden Ansprüche, **dadurch gekennzeichnet, dass** der Absolutwert der Differenz zwischen der Wellenlänge (λ₁, λ₂) des Primärlichts (3) und der Abfragewellenlänge (λ₃) größer als 20cm⁻¹ ist.

14. Verfahren nach einem der vorangehenden Ansprüche, **dadurch gekennzeichnet, dass** mindestens ein notch-Filter (5, 8) zum Eliminieren von Rayleigh-Streuung verwendet wird.

15. Verfahren nach Anspruch 1 oder 2, **dadurch gekennzeichnet, dass** im wesentlichen zeitgleich mit dem Primärlicht (3) ein Anregungslicht mit einer Wellenlänge (λ_{Anr}) in das Körpergewebe (1) eingestrahlt wird, die von der Substanz absorbiert wird.

16. Verfahren nach einem der vorangehenden Ansprüche, **dadurch gekennzeichnet, dass** die Wellenlänge (λ_{Anr}) des Anregungslichtes zwischen 1,2µm und 3µm liegt.

17. Verfahren nach einem der vorangehenden Ansprüche, **dadurch gekennzeichnet, dass** zum Eichen der Konzentrationsbestimmung der gesuchten Substanz die Intensität des ersten Vergleichssignals bei der wenigstens einen Abfragewellenlänge (λ₃) mit einer Intensität des ersten und/oder zweiten Raman-Spektralsignals bei einer weiteren Abfragewellenlänge (λ₄) verglichen wird.

18. Vorrichtung zum nicht-invasiven Bestimmen einer Konzentration einer Substanz in einer Körperflüssigkeit in einem Körpergewebe (1) mit Raman-Spektroskopie mit:
a) einer ersten Lichtquelle (2) zum Einstrahlen von monochromatischem Primärlicht (3) einer ersten Wellenlänge (λ₁) in das Körpergewebe,
b) einer Photodetektoreinrichtung (9, 11) zum Erfassen von Sekundärlicht (7), das von dem Körpergewebe zurückgestreut wird,
c) einem ersten Speicher (12) zum Abspeichern der Intensität des Sekundärlichts (7) in Abhängigkeit von der Wellenlänge als erstes Raman-Spektralsignal bei der ersten Wellenlänge (λ₁),
d) einer zweiten Lichtquelle (13) zum Einstrahlen von monochromatischem Primärlicht (3) einer zweiten Wellenlänge (λ₂) in das Körpergewebe,
e) einem zweiten Speicher (19) zum Abspeichern der Intensität des Sekundärlichts (7) in Abhängigkeit von der Wellenlänge als zweites Raman-Spektralsignal bei der zweiten Wellenlänge (λ₂),
f) einer Komparatoreinrichtung (20) zum Vergleichen des ersten Raman-Spektralsignals in dem ersten Speicher (12) und des zweiten Raman-Spektralsignals in dem zweiten Speicher (19) miteinander und zum Erzeugen eines Vergleichssignals, bei dem spektrale Strukturen, die durch das Körpergewebe bedingt sind, im wesentlichen vollständig eliminiert sind,
g) einer Diskriminatoreinheit (21) zum Bestimmen einer Intensität des Vergleichssignals bei wenigstens einer Abfragewellenlänge (λ₃), wobei die Intensität der Konzentration der Substanz in der Körperflüssigkeit entspricht,
wobei die erste (2) und/oder zweite Lichtquelle (13), die Photodetektoreinrichtung (9, 11) und der erste (12) und der zweite (19) Speicher mit einer vorgegebenen Frequenz pulsbar sind.

19. Vorrichtung zum nicht-invasiven Bestimmen einer Konzentration einer Substanz in einer Körperflüssigkeit in einem Körpergewebe (1) mit Raman-Spektroskopie mit:
a) einer Lichtquelle (2) zum Einstrahlen von monochromatischem Primärlicht (3) einer vorgegebenen Wellenlänge (λ) in das Körpergewebe,
b) einer Heizeinrichtung (25) zum Einstellen einer ersten Temperatur und einer zweiten Temperatur in dem Körpergewebe,
c) einer Photodetektoreinrichtung (9, 11) zum Erfassen von Sekundärlicht (7), das von dem Körpergewebe zurückgestreut wird,
d) einem ersten Speicher (12) zum Abspeichern der Intensität des Sekundärlichts (7) in Abhängigkeit von der Wellenlänge als erstes Raman-Spektralsignal bei der ersten Temperatur und einem zweiten Speicher (19) zum Abspeichern der Intensität des Sekundärlichts (7) in Abhängigkeit von der Wellenlänge als zweites Raman- Spektralsignal bei der zweiten Temperatur,
e) einer Komparatoreinrichtung (20) zum Vergleichen des ersten Raman-Spektralsignals in dem ersten Speicher (12) und des zweiten Raman-Spektralsignals in dem zweiten Speicher (19) miteinander und zum Erzeugen eines Vergleichssignals, bei dem spektrale Strukturen, die durch das Körpergewebe bedingt sind, im wesentlichen vollständig eliminiert sind,
f) einer Diskriminatoreinrichtung (21) zum Bestimmen einer Intensität des Vergleichssignals bei wenigstens einer Abfragewellenlänge (λ₃), wobei die Intensität der Konzentration der Substanz in der Körperflüssigkeit entspricht.

20. Vorrichtung zum nicht-invasiven Nachweis einer Substanz in einer Körperflüssigkeit in einem Körpergewebe (1) mit Raman-Spektroskopie mit:
a) einer ersten Lichtquelle (2) zum Einstrahlen von monochromatischem Primärlicht (3) einer ersten Wellenlänge (λ₁) in das Körpergewebe,
b) einer zweiten Lichtquelle (13) zum Einstrahlen von monochromatischem Primärlicht (3) einer zweiten Wellenlänge (λ₂) in das Körpergewebe,
c) einer Heizeinrichtung (25) zum Einstellen einer ersten Temperatur und einer zweiten Temperatur in dem Körpergewebe,
d) einer Photodetektoreinrichtung (9, 11) zum Erfassen von Sekundärlicht (7), das von dem Körpergewebe zurückgestreut wird,
e) einem ersten Speicher (12) zum Abspeichern der Intensität des Sekundärlichts (7) in Abhängigkeit von der Wellenlänge als erstes Raman-Spektralsignal bei der ersten Wellenlänge (λ₁) und bei der ersten Temperatur, einem zweiten Speicher (19) zum Abspeichern der Intensität des Sekundärlichts (7) in Abhängigkeit von der Wellenlänge als zweites Raman- Spektralsignal bei der zweiten Wellenlänge (λ₂) und bei der ersten Temperatur, einem dritten Speicher (26) zum Abspeichern der Intensität des Sekundärlichts (7) in Abhängigkeit von der Wellenlänge als drittes Raman-Spektralsignal bei der ersten Wellenlänge (λ₁) und bei der zweiten Temperatur und einem vierten Speicher (27) zum Abspeichern der Intensität des Sekundärlichts (7) in Abhängigkeit von der Wellenlänge als viertes Raman- Spektralsignal bei der zweiten Wellenlänge (λ₂) und bei der zweiten Temperatur,
f) einer ersten Komparatoreinrichtung (20) zum Vergleichen des ersten Raman-Spektralsignals in dem ersten Speicher (12) und des zweiten Raman-Spektralsignals in dem zweiten Speicher (19) miteinander und zum Erzeugen eines ersten Vergleichssignals, bei dem spektrale Strukturen, die durch das Körpergewebe bedingt sind, im wesentlichen vollständig eliminiert sind,
g) einer zweiten Komparatoreinrichtung (28) zum Vergleichen des dritten Raman-Spektralsignals in dem dritten Speicher (26) und des vierten Raman-Spektralsignals in dem vierten Speicher (27) miteinander und zum Erzeugen eines zweiten Vergleichssignals, bei dem spektrale Strukturen, die durch das Körpergewebe bedingt sind, im wesentlichen vollständig eliminiert sind,
h) einer dritten Komparatoreinrichtung (29) zum Vergleichen des ersten Vergleichssignals und zweiten Vergleichssignals miteinander und zum Erzeugen eines dritten Vergleichssignals in Abhängigkeit von dem Vergleichsergebnis,
i) einer Diskriminatoreinrichtung (21) zum Bestimmen einer Intensität des dritten Vergleichssignals bei wenigstens einer Abfragewellenlänge (λ₃),
j) einer Ausgabeeinheit (22) zum Ausgeben eines Identifikationssignals in Abhängigkeit von der Intensität bei der wenigstens einen Abfragewellenlänge (λ₃)

21. Vorrichtung nach Anspruch 20, **dadurch gekennzeichnet, dass** die erste (2) und/oder zweite Lichtquelle (13), die Photodetektoreinrichtung (9, 11) und der erste (12) bzw. dritte (26) und/oder zweite (19) bzw. vierte (27) Speicher mit einer vorgegebenen Frequenz pulsbar sind.

22. Vorrichtung nach Anspruch 21, **dadurch gekennzeichnet, dass** die vorgegebene Frequenz im Bereich von einigenkHz liegt.

23. Vorrichtung nach Anspruch 19 oder 20, **dadurch gekennzeichnet, dass** die Heizvorrichtung (25) zum Einstellen der ersten und zweiten Temperatur des Körpergewebes mit einer vorgegebenen Frequenz angesteuert wird.

24. Vorrichtung nach Anspruch 23, **dadurch gekennzeichnet, dass** eine Aufnahmevorrichtung zum Erfassen der Herzfrequenz vorgesehen ist und dass die vorgegebene Frequenz die Herzfrequenz ist.

25. Vorrichtung nach einem der Ansprüche 18 bis 24, **dadurch gekennzeichnet, dass** die erste (2) und/oder zweite Lichtquelle (13) Pulse mit einer Pulslänge im Pikosekundenbereich erzeugt.

26. Vorrichtung nach Anspruch 25, **dadurch gekennzeichnet, dass** die erste (2) und/oder zweite Lichtquelle (13) ein Laser ist.

27. Vorrichtung nach einem der Ansprüche 18 bis 26, **dadurch gekennzeichnet, dass** die erste (2) und/oder zweite Lichtquelle (13) eine Wellenlänge (λ₁, λ₂) des Primärlichtes (3) zwischen 750nm und 850nm erzeugt.

28. Vorrichtung nach einem der Ansprüche 18 bis 27, **gekennzeichnet durch** mindestens ein notch-Filter (4, 5, 8) zum Eliminieren von Rayleigh-Streuung, das zwischen der Abbildungsoptik (6) und der Photodetektoreinrichtung (9, 11) angeordnet ist.

29. Vorrichtung: nach einem der Ansprüche 18 bis 28, **gekennzeichnet durch** eine dritte Lichtquelle (24) zum Erzeugen von Anregungslicht mit einer Wellenlänge (λ_{Anr}), die von der Substanz absorbiert wird.

30. Vorrichtung nach Anspruch 29, **dadurch gekennzeichnet, dass** die dritte Lichtquelle (24) eine Wellenlänge (λ_{Anr}) zwischen 1,2µm und 3µm erzeugt.

31. Vorrichtung nach Anspruch 29 oder 30, **dadurch gekennzeichnet, dass** die dritte Lichtquelle (24) ein Laser für die Erzeugung von Lichtpulsen im Pikosekundenbereich ist.

32. Verfahren zum nicht- invasiven Nachweis einer Substanz in einer Körperflüssigkeit in einem Körpergewebe (1) mit Raman-Spektroskopie, das die Schritte umfasst:
a) Einstrahlen von monochromatischem Primärlicht (3) einer ersten Primärwellenlänge (λ₁) in das Körpergewebe (1) bei einem ersten Parameterwert,
b) Erfassen von Sekundärlicht (7), das von dem Körpergewebe zurückgestreut wird, und Abspeichern der Intensität des Sekundärlichts in Abhängigkeit von der Wellenlänge als erstes Raman-Spektralsignal bei der ersten Primärwellenlänge (λ₁) und bei dem ersten Parameterwert in einem ersten Speicher (12),
c) Wiederholen der Schritte a) und b) bei weiteren Primärwellenlängen und weiteren Parameterwerten, wobei die Intensität des Sekundärlichtes (7) bei jeder weiteren Primärwellenlänge und bei jedem weiteren Parameterwert in einem jeweils weiteren Speicher (26) abgespeichert wird,
d) multivariates statistisches Vergleichen der Raman- Spektralsignale zum Bestimmen wenigstens einer Abfragewellenlänge (λ₃), bei der sich eine Intensität analog zu den Parameterwerten ändert, und
e) Bestimmen und Ausgeben der Intensität bei der wenigstens einen Abfragewellenlänge (λ₃) in den mehreren Raman- Spektralsignalen als Funktion der Parameterwerte.

33. Verfahren nach dem vorangehenden Anspruch, bei dem der Parameter ein Blutzuckerspiegel ist.

## Claims

1. A method for the non-invasive determination of a concentration of a substance in a body fluid in a body tissue (1) by Raman spectroscopy comprising the following steps:
a) irradiating monochromatic primary light (3) of a first wavelength (λ₁) into the body tissue (1),
b) recording secondary light (7) that is back scattered by the body tissue and storing the intensity of the secondary light in a first memory (12) according to its wavelength as a first Raman spectral signal for the first primary wavelength (λ₁),
c) irradiating monochromatic primary light (3) of a second wavelength (λ₂) into the body tissue (1),
d) recording secondary light (7) that is back scattered by the body tissue and storing the intensity of the secondary light in a second memory (19) according to its wavelength as a second Raman spectral signal for the second primary wavelength (λ₂),
e) comparing the first Raman spectral signal in the first memory (12) with the second Raman spectral signal in the second memory (19) and generating a compare signal in which spectral structures caused by the body tissue are substantially eliminated,
f) determining an intensity of the compare signal at at least one sample wavelength (λ₃) for which the intensity corresponds to the concentration of the substance in the body fluid, the primary light (3) of the first and/or second wavelength is irradiated with a preset irradiation frequency and the first and second Raman spectral signals are recorded with the irradiation frequency.

2. A method for non-invasive determination of a concentration of a substance in a body tissue (1) by Raman spectroscopy comprising the following steps:
a) irradiating monochromatic primary light (3) of a given wavelength (A) into the body tissue (1) at a first temperature of the body tissue,
b) recording secondary light (7) that is back scattered by the body tissue and storing the intensity of the secondary light in a first memory (12) according to its wavelength as a first Raman spectral signal for the first temperature,
c) irradiating monochromatic primary light (3) of said given wavelength (λ) into the body tissue (1) at a second temperature of the body tissue,
d) recording secondary light (7) that is back scattered from the body tissue and storing the intensity of the secondary light in a second memory (19) according to its wavelength as a second Raman spectral signal for the second temperature,
e) comparing the first Raman spectral signal in the first memory (12) with the second Raman spectral signal in the second memory (19) and generating a compare signal for which spectral structures caused by the body tissue are substantially eliminated,
f) determining an intensity of the compare signal at at least one sample wavelength (λ₃) for which the intensity corresponds to the concentration of the substance in the body fluid.

3. A method for non-invasive determination of a concentration of a substance in a body tissue (1) by Raman spectroscopy comprising the following steps:
a) irradiating monochromatic primary light (3) of a first wavelength (λ₁) into the body tissue (1) at a first temperature of the body tissue,
b) recording secondary light (7) that is back scattered by the body tissue and storing the intensity of the secondary light in a first memory (12) according to its wavelength as a first Raman spectral signal for the first wavelength (λ₁) and for the first temperature,
c) irradiating monochromatic primary light (3) of a second wavelength (λ₂) into the body tissue (1) at a first temperature of the body tissue,
d) recording secondary light (7) that is back scattered by the body tissue and storing the intensity of the secondary light in a second memory (19) according to its wavelength as a second Raman spectral signal for the second wavelength (λ₂) and for the first temperature,
e) comparing the first Raman spectral signal in the first memory (12) with the second Raman spectral signal in the second memory (19) and generating a compare signal for which spectral structures caused by the body tissue are substantially eliminated,
f) determining an intensity of the compare signal at at least one sample wavelength(λ₃),
g) repeating steps a) to f) for a second temperature and generating a second compare signal at at least one sample wavelength (λ₃) for which the intensities of the secondary light (7) for the first and second wavelengths (λ₁, λ₂) are stored in a third and a fourth memory (26, 28), respectively, and a second compare signal is generated,
h) comparing the first and the second compare signal and, depending on the result of this comparison, generating an identification signal at at least one sample wavelength (λ₃).

4. The method according to claim 3, wherein the primary light (3) of the first and/or second wavelength is irradiated with a preset irradiation frequency and the first and second Raman spectral signals are recorded with the irradiation frequency.

5. The method according to claim 4, wherein the irradiation frequency is in the range of some kHz.

6. The method according to claim 2 or 3, wherein the first and the second temperature of the body tissue is adjusted with a preset temperature variation frequency.

7. The method according to claim 6, wherein the temperature variation frequency is the heart rate.

8. The method according to any of the preceding claims, wherein the primary light (3) of the first and/or second wavelength is pulsed with a pulse length in the order of a picosecond.

9. The method according to claim 8, wherein the primary light is generated by a laser.

10. The method according to any of the preceding claims, wherein the secondary light (7) is recorded in the Stokes- and/or in the anti- Stokes region of the Raman- spectrum.

11. The method according to any of the preceding claims, wherein the wavelength (λ₁, λ₂) of the primary light (3) is in the range between 750nm and 850nm.

12. The method according to any of the preceding claims, wherein the modulus of the difference of the primary light (3) wavelength (λ₁, λ₂) and the sample wavelength (λ₃) is less than 2000cm⁻¹.

13. The method according to any of the preceding claims, wherein the modulus of the difference of the primary light (3) wavelength (λ₁, λ₂) and the sample wavelength (λ₃) is greater than 20cm⁻¹.

14. The method according to any of the preceding claims, wherein at least one notch-filter (5, 8) is used for eliminating Rayleigh scattering.

15. The method according to claim 1 or 2, wherein an excitation light wavelength (A_{Anr}) is irradiated into the body tissue (1) substantially at the same time as the primary light (3) that is absorbed by the sought after substance.

16. The method according to any of the preceding claims, wherein the excitation wavelength (λ_{Anr}) is in the range between 1.2µm and 3µm.

17. The method according to any of the preceding claims, wherein for calibrating the concentration analysis of the sought after substance the intensity of the first compare signal at the at least one sample wavelength (λ₃) is compared to an intensity of the first and/or second Raman spectral signal at another sample wavelength (λ₄).

18. A device for the non-invasive determination of a concentration of a substance in a body fluid in a body tissue (1) by Raman spectroscopy comprising:
a) a first light source (2) for irradiating monochromatic primary light (3) of a first wavelength (λ₁) into the body tissue,
b) a photo detector means (9, 11) for recording secondary light that is backscattered from the body tissue,
c) a first memory (12) for storing the intensity of the secondary light (7) according to its wavelength as a first Raman spectral signal for the first wavelength (λ₁),
d) a second light source (13) for irradiating monochromatic primary light (3) of a second wavelength (λ₂) into the body tissue,
e) a second memory (19) for storing the intensity of the secondary light (7) according to its wavelength as a second Raman spectral signal for the second wavelength (λ₂),
f) a comparator means (20) for comparing the first Raman spectral signal in the first memory (12) with the second Raman spectral signal in the second memory (19) and for generating a compare signal for which spectral structures caused by the body tissue are substantially eliminated,
g) a discriminator unit (21) for determining an intensity of the compare signal at at least one sample wavelength (λ₃) for which the intensity corresponds to the concentration of the substance in the body fluid.
wherein the first (2) and/or second (13) light source, the photo-detector means (9, 11) and the first (12) and the second memory (19) are pulsed with a preset irradiation frequency.

19. A device for the non-invasive determination of a concentration of a substance in a body fluid in a body tissue (1) by Raman spectroscopy comprising:
a) a light source (2) for irradiating monochromatic primary light (3) of a given wavelength (A) into the body tissue,
b) a heating means (25) for adjusting a first temperature and a second temperature in the body tissue,
c) a photo detector means (9, 11) for recording secondary light (7) that is backscattered from the body tissue,
d) a first memory (12) for storing the intensity of the secondary light (7) according to its wavelength as a first Raman spectral signal for the first temperature and a second memory (19) for storing the intensity of the secondary light (7) according to its wavelength as a second Raman spectral signal for the second temperature,
e) a comparator means (20) for comparing the first Raman spectral signal in the first memory (12) with the second Raman spectral signal in the second memory (19) and for generating a compare signal for which spectral structures caused by the body tissue are substantially eliminated,
f) a discriminator unit (21) for determining an intensity of the compare signal at at least one sample wavelength (λ₃) for which the intensity corresponds to the concentration of the substance in the body fluid.

20. A device for the non-invasive determination of a concentration of a substance in a body fluid in a body tissue (1) by Raman spectroscopy comprising:
a) a first light source (2) for irradiating monochromatic primary light (3) of a first wavelength (λ₁) into the body tissue,
b) a second light source (13) for irradiating monochromatic primary light (3) of a second wavelength (λ₂) into the body tissue,
c) a heating means (25) for adjusting a first temperature and a second temperature in the body tissue,
d) a photo detector means (9, 11) for recording secondary light (7) that is backscattered from the body tissue,
e) a first memory (12) for storing the intensity of the secondary light (7) according to its wavelength as a first Raman spectral signal for the first wavelength (λ₁) and for the first temperature, a second memory (19) for storing the intensity of the secondary light (7) according to its wavelength as a second Raman spectral signal for the second wavelength (λ₂) and for the first temperature, a third memory (26) for storing the intensity of the secondary light (7) according to its wavelength as a third Raman spectral signal for the first wavelength (λ₁) and for the second temperature and a fourth memory (27) for storing the intensity of the secondary light (7) according to its wavelength as a fourth Raman spectral signal for the second wavelength (λ₂) and for the second temperature,
f) a first comparator means (20) for comparing the first Raman spectral signal in the first memory (12) with the second Raman spectral signal in the second memory (19) and for generating a compare signal for which spectral structures caused by the body tissue are substantially eliminated,
g) a second comparator means (28) for comparing the third Raman spectral signal in the third memory (26) with the fourth Raman spectral signal in the fourth memory (27) and for generating a compare signal for which spectral structures caused by the body tissue are substantially eliminated,
h) a third comparator means (29) for comparing the first compare signal with the second compare signal for generating a third compare signal depending of the result of this comparison,
i) a discriminator unit (21) for determining an intensity of the third compare signal at at least one sample wavelength (λ₃),
j) an output unit (22) for the output of an identification signal according to the intensity at at least one sample wavelength (λ₃) for which the intensity corresponds to the concentration of the substance in the body fluid.

21. The device according to claim 20, wherein the first (2) and/or second light source (13), the photo-detector means (9, 11) and the first (12) and third (26), respectively, and/or second (19) and fourth (27) memory, respectively, is/are pulsed with a preset irradiation frequency.

22. The method according to claim 21, wherein the irradiation frequency is in the range of some kHz.

23. The device according to claim 19 or 20, wherein the heating means (25) for adjusting the first and second temperature of the body tissue is triggered with a preset temperature variation frequency.

24. The device according to claim 23, wherein a receiver means is provided for recording the heart rate and the temperature variation frequency is the heart rate.

25. The device according to any of claims 18 to 24, wherein the first (2) and/or second light source (13) generates pulses of a pulse length in the picosecond region.

26. The device according to claim 25, wherein the first (2) and/or the second primary light source (13) is a laser.

27. The device according to any of claims 18 to 26, wherein the first (2) and/or second light source (13) generates a primary light (3) wavelength (λ₁, λ₂) between 750nm and 850nm.

28. The device according to any of claims 18 to 27, wherein at least one notch filter (4, 5, 8) is positioned between the projection optics (6) and the photo detector assembly (9, 11) to eliminate Rayleigh scattering.

29. The device according to any of claims 18 to 28, wherein a third light source (24) is provided for generating an excitation light wavelength (λ_{Anr}) which is absorbed by the substance.

30. The device according to claim 29, wherein the third light source (24) generates light of a wavelength (λ_{Anr}) between 1.2µm and 3µm.

31. The device according to any of claims 29 or 30, wherein the third light source (24) is a laser for generating picosecond pulses.

32. A method for the non-invasive determination of a concentration of a substance in a body fluid in a body tissue (1) by Raman spectroscopy comprising the following steps:
a) irradiating monochromatic primary light (3) of a first wavelength (λ₁) into the body tissue (1) at a first parameter value,
b) recording secondary light (7) that is back scattered by the body tissue and storing the intensity of the secondary light in a first memory (12) according to its wavelength as a first Raman spectral signal for the first primary wavelength (λ₁) and for the first parameter value,
c) repeating steps a) and b) for other primary wavelengths and other parameter values for which the intensity of the secondary light (7) is stored in a further memory (26) for each further primary wavelength and for each further parameter value,
d) multivariate statistical comparison of the Raman signals for the determining at least one sample wavelength (λ₃) for which the intensity changes analogously to the parameter values, and
e) determining and outputting the intensity at at least one sample wavelength (λ₃) in several Raman spectral signals according to the parameter values.

33. The method according to claim 32, wherein the parameter is the blood sugar level.

## Revendications

1. Procédé de détermination non invasive d'une concentration de substance dans un liquide physiologique d'un tissu organique (1) par spectroscopie Raman, comprenant les étapes suivantes :
a) irradiation de lumière primaire monochromatique (3) d'une première longueur d'onde (λ₁) dans le tissu organique (1),
b) enregistrement de lumière secondaire (7) dispersée en retour par le tissu organique et sauvegarde de l'intensité de la lumière secondaire en fonction de la longueur d'onde sous forme de premier signal spectral Raman pour la première longueur d'onde (λ₁) dans une première mémoire (12),
c) irradiation de lumière primaire monochromatique (3) d'une deuxième longueur d'onde (λ₂) dans le tissu organique (1),
d) enregistrement de lumière secondaire (7) dispersée en retour par le tissu organique et sauvegarde de l'intensité de la lumière secondaire en fonction de la longueur d'onde sous forme de deuxième signal spectral Raman pour la deuxième longueur d'onde (λ₂) dans une deuxième mémoire (12),
e) comparaison l'un à l'autre du premier signal spectral Raman de la première mémoire (12) et du deuxième signal spectral Raman de la deuxième mémoire (19) et génération d'un signal comparatif avec lequel les structures spectrales dues au tissu organique sont sensiblement éliminées totalement,
f) détermination d'une intensité du signal comparatif pour au moins une longueur d'onde d'interrogation (λ₃), l'intensité correspondant à la concentration de substance dans le liquide physiologique, tandis que la lumière primaire (3) de première et/ou deuxième longueur d'onde est irradiée à une fréquence prescrite et que le premier et le deuxième signal spectral Raman sont relevés à la fréquence prescrite.

2. Procédé de détermination non invasive d'une concentration de substance dans un liquide physiologique d'un tissu organique (1) par spectroscopie Raman, comprenant les étapes suivantes :
a) irradiation de lumière primaire monochromatique (3) d'une longueur d'onde prescrite (λ) dans le tissu organique (1) à une première température du tissu organique,
b) enregistrement de lumière secondaire (7) dispersée en retour par le tissu organique et sauvegarde de l'intensité de la lumière secondaire en fonction de la longueur d'onde sous forme de premier signal spectral Raman à la première température dans une première mémoire (12),
c) irradiation de lumière primaire monochromatique (3) de la longueur d'onde prescrite (λ) à une deuxième température du tissu organique,
d) enregistrement de lumière secondaire (7) dispersée en retour par le tissu organique et sauvegarde de l'intensité de la lumière secondaire en fonction de la longueur d'onde sous forme de deuxième signal spectral Raman à la deuxième température dans une deuxième mémoire (19),
e) comparaison l'un à l'autre du premier signal spectral Raman de la première mémoire (12) et du deuxième signal spectral Raman de la deuxième mémoire (19) et génération d'un signal comparatif avec lequel les structures spectrales dues au tissu organique sont sensiblement éliminées totalement,
f) détermination d'une intensité du signal comparatif pour au moins une longueur d'onde d'interrogation (λ₃), l'intensité correspondant à la concentration de substance dans le liquide physiologique.

3. Procédé de mise en évidence non invasive d'une substance dans un liquide physiologique d'un tissu organique (1) par spectroscopie Raman, comprenant les étapes suivantes :
a) irradiation de lumière primaire monochromatique (3) d'une première longueur d'onde (λ₁) dans le tissu organique (1) à une première température,
b) enregistrement de lumière secondaire (7) dispersée en retour par le tissu organique et sauvegarde de l'intensité de la lumière secondaire en fonction de la longueur d'onde sous forme de premier signal spectral Raman à la première longueur d'onde (λ₁) et à la première température dans une première mémoire (12),
c) irradiation de lumière primaire monochromatique (3) d'une deuxième longueur d'onde (λ₂) dans le tissu organique (1) à une première température,
d) enregistrement de lumière secondaire (7) dispersée en retour par le tissu organique et sauvegarde de l'intensité de la lumière secondaire en fonction de la longueur d'onde sous forme de deuxième signal spectral Raman à la deuxième longueur d'onde (λ₂) et à la première température dans une deuxième mémoire (19),
e) comparaison l'un à l'autre du premier signal spectral Raman de la première mémoire (12) et du deuxième signal spectral Raman de la deuxième mémoire (19) et génération d'un premier signal comparatif avec lequel les structures spectrales dues au tissu organique sont sensiblement éliminées totalement,
f) détermination d'une intensité du premier signal comparatif pour au moins une longueur d'onde d'interrogation (λ₃),
g) répétition des étapes a) à f) à une deuxième température et génération d'un deuxième signal comparatif pour l'au moins une longueur d'onde d'interrogation (λ₃), l'intensité de la lumière secondaire (7) pour la première et la deuxième longueur d'onde (λ₁, λ₂) étant sauvegardée dans une troisième mémoire (26) ou une quatrième mémoire (28) et un deuxième signal comparatif étant généré,
h) comparaison du premier et du deuxième signal comparatif et génération d'un signal d'identification en fonction du résultat de la comparaison pour l'au moins une longueur d'onde d'interrogation (λ₃).

4. Procédé selon la revendication 3, **caractérisé en ce que** la lumière primaire (3) de première et/ou deuxième longueur d'onde est irradiée à une fréquence prescrite et que le premier et le deuxième signal spectral Raman sont relevés à la fréquence prescrite.

5. Procédé selon la revendication 4, **caractérisé en ce que** la fréquence prescrite se situe dans la plage de quelques kHz.

6. Procédé selon la revendication 2 ou 3, **caractérisé en ce que** la première et la deuxième température du tissu organique sont établies à une fréquence prescrite.

7. Procédé selon la revendication 6, **caractérisé en ce que** la fréquence prescrite est la fréquence cardiaque.

8. Procédé selon une des revendications précédentes, **caractérisé en ce que** la lumière primaire (3) à la première et/ou deuxième longueur d'onde présente une longueur d'impulsion de l'ordre des picosecondes.

9. Procédé selon la revendication 8, **caractérisé en ce que** la lumière primaire est générée par un laser.

10. Procédé selon une des revendications précédentes, **caractérisé en ce que** la lumière secondaire (7) est enregistrée dans la plage Stokes et/ou la plage anti-Stokes du spectre Raman.

11. Procédé selon une des revendications précédentes, **caractérisé en ce que** la longueur d'onde (λ₁, λ₂) de la lumière primaire (3) se situe entre 750 nm et 850 nm.

12. Procédé selon une des revendications précédentes, **caractérisé en ce que** la valeur absolue de la différence entre la longueur d'onde (λ₁, λ₂) de la lumière primaire (3) et la longueur d'onde d'interrogation (λ₃) est inférieure à 2000 cm⁻¹.

13. Procédé selon une des revendications précédentes, **caractérisé en ce que** la valeur absolue de la différence entre la longueur d'onde (λ₁, λ₂) de la lumière primaire (3) et la longueur d'onde d'interrogation (λ₃) est supérieure à 20 cm⁻¹.

14. Procédé selon une des revendications précédentes, **caractérisé en ce qu'**au moins un filtre à bande d'arrêt étroite (5, 8) est employé pour éliminer la dispersion Rayleigh.

15. Procédé selon la revendication 1 ou 2, **caractérisé en ce que**, sensiblement en même temps que la lumière primaire (3), une lumière d'excitation d'une longueur d'onde (λ_{Anr}) est irradiée dans le tissu organique (1), cette lumière étant absorbée par la substance.

16. Procédé selon une des revendications précédentes, **caractérisé en ce que** la longueur d'onde (λ_{Anr}) de la lumière d'excitation se situe entre 1,2 µm et 3 µm.

17. Procédé selon une des revendications précédentes, **caractérisé en ce que**, pour étalonner la détermination de concentration de la substance recherchée, l'intensité du premier signal comparatif pour l'au moins une longueur d'interrogation (λ₃) est comparée à l'intensité du premier et/ou deuxième signal spectral Raman pour une autre longueur d'onde d'interrogation (λ₄).

18. Dispositif de détermination non invasive d'une concentration de substance dans un liquide physiologique d'un tissu organique (1) par spectroscopie Raman, comprenant :
a) une première source lumineuse (2) pour irradier de la lumière primaire monochromatique (3) d'une première longueur d'onde (λ₁) dans le tissu organique,
b) un dispositif photodétecteur (9, 11) pour enregistrer de la lumière secondaire (7) dispersée en retour par le tissu organique,
c) une première mémoire (12) pour sauvegarder l'intensité de la lumière secondaire (7) en fonction de la longueur d'onde sous forme de premier signal spectral Raman pour la première longueur d'onde (λ₁),
d) une deuxième source lumineuse (13) pour irradier de la lumière primaire monochromatique (3) d'une deuxième longueur d'onde (λ₂) dans le tissu organique,
e) une deuxième mémoire (19) pour sauvegarder l'intensité de la lumière secondaire (7) en fonction de la longueur d'onde sous forme de deuxième signal spectral Raman pour la deuxième longueur d'onde (λ₂),
f) un dispositif comparateur (20) pour comparer l'un à l'autre le premier signal spectral Raman de la première mémoire (12) et le deuxième signal spectral Raman de la deuxième mémoire (19) et pour générer un signal comparatif avec lequel les structures spectrales dues au tissu organique sont sensiblement éliminées totalement,
g) une unité discriminante (21) pour déterminer une intensité du signal comparatif pour au moins une longueur d'onde d'interrogation (λ₃), l'intensité correspondant à la concentration de la substance dans le liquide physiologique,
la première (2) et/ou la deuxième source lumineuse (13), le dispositif photodétecteur (9, 11) et la première (12) et la deuxième (19) mémoire pouvant être impulsées à une fréquence prescrite.

19. Dispositif de détermination non invasive d'une concentration de substance dans un liquide physiologique d'un tissu organique (1) par spectroscopie Raman, comprenant :
a) une source lumineuse (2) pour irradier de la lumière primaire monochromatique (3) d'une longueur d'onde prescrite (λ) dans le tissu organique,
b) un dispositif de chauffage (25) pour établir une première température et une deuxième température dans le tissu organique,
c) un dispositif photodétecteur (9, 11) pour enregistrer de la lumière secondaire (7) dispersée en retour par le tissu organique,
d) une première mémoire (12) pour sauvegarder l'intensité de la lumière secondaire (7) en fonction de la longueur d'onde sous forme de premier signal spectral Raman à la première température et une deuxième mémoire (19) pour sauvegarder l'intensité de la lumière secondaire (7) en fonction de la longueur d'onde sous forme de deuxième signal spectral Raman à la deuxième température,
e) un dispositif comparateur (20) pour comparer l'un à l'autre le premier signal spectral Raman de la première mémoire (12) et le deuxième signal spectral Raman de la deuxième mémoire (19) et pour générer un signal comparatif avec lequel les structures spectrales dues au tissu organique sont sensiblement éliminées totalement,
f) une unité discriminante (21) pour déterminer une intensité du signal comparatif pour au moins une longueur d'onde d'interrogation (λ₃), l'intensité correspondant à la concentration de la substance dans le liquide physiologique.

20. Dispositif de mise en évidence non invasive d'une substance dans un liquide physiologique d'un tissu organique (1) par spectroscopie Raman, comprenant :
a) une première source lumineuse (2) pour irradier de la lumière primaire monochromatique (3) d'une première longueur d'onde (λ₁) dans le tissu organique,
b) une deuxième source lumineuse (13) pour irradier de la lumière primaire monochromatique (3) d'une deuxième longueur d'onde prescrite (λ₂) dans le tissu organique,
c) un dispositif de chauffage (25) pour établir une première température et une deuxième température dans le tissu organique,
d) un dispositif photodétecteur (9, 11) pour enregistrer de la lumière secondaire (7) dispersée en retour par le tissu organique,
e) une première mémoire (12) pour sauvegarder l'intensité de la lumière secondaire (7) en fonction de la longueur d'onde sous forme de premier signal spectral Raman pour la première longueur d'onde (λ₁) et à la première température, une deuxième mémoire (19) pour sauvegarder l'intensité de la lumière secondaire (7) en fonction de la longueur d'onde sous forme de deuxième signal spectral Raman pour la deuxième longueur d'onde (λ₂) et à la première température, une troisième mémoire (26) pour sauvegarder l'intensité de la lumière secondaire (7) en fonction de la longueur d'onde sous forme de troisième signal spectral Raman pour la première longueur d'onde (λ₁) et à la deuxième température et une quatrième mémoire (27) pour sauvegarder l'intensité de la lumière secondaire (7) en fonction de la longueur d'onde sous forme de quatrième signal spectral Raman à la deuxième longueur d'onde (λ₂) et à la deuxième température,
f) un premier dispositif comparateur (20) pour comparer l'un à l'autre le premier signal spectral Raman de la première mémoire (12) et le deuxième signal spectral Raman de la deuxième mémoire (19) et pour générer un premier signal comparatif avec lequel les structures spectrales dues au tissu organique sont sensiblement éliminées totalement,
g) un deuxième dispositif comparateur (28) pour comparer l'un à l'autre le troisième signal spectral Raman de la troisième mémoire (26) et le quatrième signal spectral Raman de la quatrième mémoire (27) et pour générer un deuxième signal comparatif avec lequel les structures spectrales dues au tissu organique sont sensiblement éliminées totalement,
h) un troisième dispositif comparateur (29) pour comparer l'un à l'autre le premier signal comparatif et le deuxième signal comparatif et pour générer un troisième signal comparatif en fonction du résultat de la comparaison,
i) une unité discriminante (21) pour déterminer une intensité du troisième signal comparatif pour au moins une longueur d'interrogation (λ₃),
j) une unité d'affichage (22) pour afficher un signal d'identification en fonction de l'intensité pour l'au moins une longueur d'onde d'interrogation (λ₃).

21. Dispositif selon la revendication 20, **caractérisé en ce que** la première (1) et/ou la deuxième source lumineuse (13), le dispositif photodétecteur (9, 11) et la première (12) ou troisième (26) et/ou deuxième (19) ou quatrième (27) mémoire peuvent être impulsés à une fréquence prescrite.

22. Dispositif selon la revendication 21, **caractérisé en ce que** la fréquence prescrite se situe dans la plage de quelques kHz.

23. Dispositif selon la revendication 19 ou 20, **caractérisé en ce que** le dispositif de chauffage (25) est activé pour établir la première et la deuxième température du tissu organique à une fréquence prescrite.

24. Dispositif selon la revendication 23, **caractérisé en ce qu'**un dispositif récepteur pour l'enregistrement de la fréquence cardiaque est prévu et que la fréquence prescrite est la fréquence cardiaque.

25. Dispositif selon une des revendications 18 à 24, **caractérisé en ce que** la première (2) et/ou la deuxième source lumineuse (13) génère des impulsions d'une longueur d'impulsion de l'ordre des picosecondes.

26. Dispositif selon la revendication 25, **caractérisé en ce que** la première (2) et/ou la deuxième source lumineuse (13) est un laser.

27. Dispositif selon une des revendications 18 à 26, **caractérisé en ce que** la première (2) et/ou la deuxième source lumineuse (13) génère une longueur d'onde (λ₁, λ₂) de la lumière primaire (3) entre 750 nm et 850 nm.

28. Dispositif selon une des revendications 18 à 27, **caractérisé par** au moins un filtre à bande d'arrêt étroite (4, 5, 8) pour éliminer la dispersion Rayleigh qui est disposé entre l'optique de reproduction (6) et le dispositif photodétecteur (9, 11).

29. Dispositif selon une des revendications 18 à 28, **caractérisé par** une troisième source lumineuse (24) pour générer de la lumière d'excitation d'une longueur d'onde (λ_{Anr}) qui est absorbée par la substance.

30. Dispositif selon la revendication 29, **caractérisé en ce que** la troisième source lumineuse (24) génère une longueur d'onde (λ_{Anr}) allant de 1,2 µm à 3 µm.

31. Dispositif selon la revendication 29 ou 30, **caractérisé en ce que** la troisième source lumineuse (24) est un laser pour la génération d'impulsions lumineuses.

32. Procédé de mise en évidence non invasive d'une substance dans un liquide physiologique d'un tissu organique (1) par spectroscopie Raman, comprenant les étapes suivantes :
a) irradiation de lumière primaire monochromatique (3) d'une première longueur d'onde (λ₁) dans le tissu organique (1) pour une première valeur paramétrique,
b) enregistrement de lumière secondaire (7) dispersée en retour par le tissu organique et sauvegarde de l'intensité de la lumière secondaire en fonction de la longueur d'onde sous forme de premier signal spectral Raman pour la première longueur d'onde (λ₁) et la première valeur paramétrique dans une première mémoire (12),
c) répétition des étapes a) et b) pour les autres longueurs d'ondes primaires et les autres valeurs paramétriques, l'intensité de la lumière secondaire (7) pour chaque autre longueur d'onde primaire et chaque autre valeur paramétrique étant sauvegardée dans une autre mémoire respective (26),
d) comparaison statistique à variables multiples des signaux spectraux Raman pour déterminer au moins une longueur d'onde d'interrogation (λ₃) pour laquelle une intensité varie de manière analogue aux valeurs paramétriques et
e) détermination et affichage de l'intensité pour l'au moins une longueur d'onde d'interrogation (λ₃) dans les plusieurs signaux spectraux Raman en fonction des valeurs paramétriques.

33. Procédé selon la revendication précédente, dans lequel le paramètre est un niveau de sucre dans le sang.
